(19) Europäisches Patentamt
European Patent Office
Office européen des brevets

(11) EP 2 535 039 A1

(12) EUROPEAN PATENT APPLICATION
published in accordance with Art. 153(4) EPC

(43) Date of publication:
**19.12.2012 Bulletin 2012/51**

(21) Application number: **11854536.7**

(22) Date of filing: **12.04.2011**

(51) Int Cl.:
*A61K 8/64* (2006.01) *A61K 8/40* (2006.01)
*A61K 8/42* (2006.01) *A61Q 5/02* (2006.01)
*A61Q 5/04* (2006.01) *A61Q 5/10* (2006.01)
*A61Q 5/12* (2006.01)

(86) International application number:
**PCT/JP2011/059045**

(87) International publication number:
**WO 2012/140725 (18.10.2012 Gazette 2012/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(71) Applicant: **Seiwa Kasei Company, Limited**
**Higashiosaka-shi**
**Osaka 579-8004 (JP)**

(72) Inventors:
- **YOSKIOKA, Masato**
  **Higashiosaka-shi**
  **Osaka 579-8004 (JP)**
- **KOBAYASHI, Eriko**
  **Higashiosaka-shi**
  **Osaka 579-8004 (JP)**
- **KASAHARA, Yoshihito**
  **Higashiosaka-shi**
  **Osaka 579-8004 (JP)**
- **TOKANO, Takao**
  **Higashiosaka-shi**
  **Osaka 579-8004 (JP)**
- **NAKAMURA, Sayaka**
  **Higashiosaka-shi**
  **Osaka 579-8004 (JP)**

(74) Representative: **Duckett, Anthony Joseph et al**
**Mathys & Squire LLP**
**120 Holborn**
**London**
**EC1N 2SQ (GB)**

# (54) COSMETIC BASE MATERIAL, AND COSMETIC CONTAINING SAID COSMETIC BASE MATERIAL

(57) A cosmetic base material composed of a peptide or peptide derivative having one or more acidic amino acid as the constituent amino acid and in which at least some of anionic functional groups thereof form an ion complex with at least one surfactant selected from the group consisting of cationic surfactants and ampholytic surfactants.



Printed by Jouve, 75001 PARIS (FR)

## Description

### Technical Field

The present invention relates to a cosmetic base material composed of a peptide or peptide derivative having an acidic amino acid as the constituent amino acid and in which at least some of anionic functional groups thereof form an ion complex with at least one surfactant selected from the group consisting of cationic surfactants and ampholytic surfactants, and to a cosmetic containing the cosmetic base material. That is, the present invention relates to a cosmetic base material composed of a specific peptide or peptide derivative having an ion complex with a specific surfactant as described above and manifesting an excellent conditioning effect on hair when compounded in a cosmetic, particularly, a hair treating agent such as a hair treatment and the like, and to a cosmetic containing the cosmetic base material.

### Background Art

On the surface of healthy hair, hydrophobic substances such as 18-methyleicosanoic acid and the like are present, and these are believed to contribute significantly to hair sensory character such as flexibility, moist feeling, luster, smoothness and the like. Hair damaged by chemical treatments such as permanent waving, hair coloration and the like and physical treatments such as brushing and the like loses hydrophobicity of the hair surface, and shows remarkably lowered sensory characters such as lowered flexibility, moist feeling, luster and smoothness. Then, various cosmetic base materials having hair conditioning effects are developed and compounded in cosmetics for improving these lowered sensory characters.

As the cosmetic base material having a hair conditioning effects, peptides and derivatives thereof are often used because of their strong adsorption force onto hair, and have already been compounded in hair treating agents such as a hair treatment, shampoo and the like. Among them, protein hydrolyzates (hydrolyzed proteins) obtained by hydrolyzing proteins, and derivatives thereof are widely used (patent document 1).

As the peptide derivative, acylated hydrolyzed proteins obtained by amide-bonding a protein hydrolyzate to a fatty acid are developed as cosmetic base materials having an enhanced hair conditioning effect of the protein hydrolyzate, and compounded in various hair treating agents (patent document 2, patent document 3).

As the surfactant having a hair conditioning effect, fatty acid amide amines and monoalkyl trimethyl ammonium salts are generally known, and these are compounded for hydrophobizing the surface of hair, of which hydrophobicity was deteriorated by damages, thereby approaching sensory characters of healthy hair (patent document 4, patent document 5, patent document 6).

There are problems, however, that though peptides and derivatives thereof show strong adsorption force onto hair, an ability of hydrophobizing hair, of which hydrophobicity was deteriorated by damages, is weak. On the other hand, only with fatty acid amide amines and monoalkyl trimethyl ammonium salts, a sufficient conditioning effect cannot be manifested because of their weak adsorption force onto damaged hair.

### Prior Art Document

### Patent Document

Patent document 1: JP-2007-302615A
Patent document 2: JP-2004-196733A
Patent document 3: JP-2004-231517A
Patent document 4: JP-06-45526B
Patent document 5: Japanese Patent No. 4247805
Patent document 6: Japanese Patent No. 3981828

### Summary of the Invention

### Problem to be solved by the Invention

The present invention has an object of providing a cosmetic base material which hydrophobizes the surface of hair, of which their sensory characters were lowered by losing the hydrophobicity of the hair surface by damages, thereby imparting flexibility, moisture retaining property, smoothness, luster and the like to hair, and a cosmetic containing the cosmetic base material.

Means for Solving the Problem

The present inventors have intensively studied to solve the above-described problem and resultantly found that an ion complex formed by ionically bonding an anionic functional group of a peptide or peptide derivative having an acidic amino acid as the constituent amino acid with at least one surfactant selected from the group consisting of cationic surfactants and ampholytic surfactants shows high adsorption force onto hair and has an excellent effect which hydrophobizes the surface of hair, of which their sensory characters were lowered by losing the hydrophobicity of the hair surface by damages, thereby imparting flexibility, moisture retaining property, smoothness, luster and the like to hair. The present invention was thus completed.

That is, the present invention provides, as a basic invention, a cosmetic base material composed of a peptide or peptide derivative having an acidic amino acid as the constituent amino acid (hereinafter, this is represented by "component A" in some cases) and in which at least some of anionic functional groups thereof form an ion complex with at least one surfactant selected from the group consisting of cationic surfactants and ampholytic surfactants (hereinafter, this is represented by "component B" in some cases). This is called as an invention according to Claim 1.

In the cosmetic base material of above-described invention, at least some of anionic functional groups of the peptide or peptide derivative having one or more acidic amino acid as the constituent amino acid are ionically bonded to the surfactant as the component B to form an ion complex. It is preferable that the ratio of anionic functional groups in the peptide or peptide derivative forming the above-described ion complex with respect to all anionic functional groups in the peptide or peptide derivative is a certain level or more from the standpoint of properties.

Thus, in the present invention, the cosmetic base material according to Claim 1 in which 30 mol% or more of all anionic functional groups in the above-described peptide or peptide derivative having an acidic amino acid as the constituent amino acid are ionically bonded to at least one surfactant selected from the group consisting of cationic surfactants and ampholytic surfactants to form an ion complex is provided as an invention according to Claim 2.

In the present invention, the anionic functional group denotes a carboxyl group and a sulfo group, and all anionic functional groups include carboxyl groups and sulfo groups on the side chain of an acidic amino acid, and terminal carboxyl groups on the main chain of the peptide. The sum of these anionic functional groups corresponds 100 mol%.

Since at least some of anionic functional groups of a peptide or peptide derivative having one or more acidic amino acid as the constituent amino acid are ionically bonded to a surfactant as the component B to form an ion complex in the cosmetic base material of the present invention, it is preferable that the surfactant as the component B is easily ionically bonded to anionic functional groups of the peptide or peptide derivative and shows high safeness even if bonded to a cosmetic.

Thus, in the present invention, the cosmetic base material according to Claim 1 or 2 in which the above-described at least one surfactant selected from the group consisting of cationic surfactants and ampholytic surfactants includes a fatty acid amide amine represented by the general formula (I):

(Chemical formula 1)

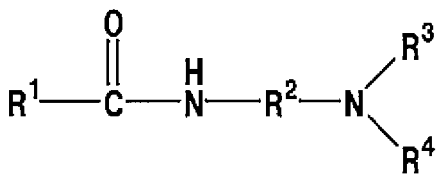

(I)

(wherein, $R^1$ represents a linear or branched hydrocarbon group having 11 to 25 carbon atoms, $R^2$ represents an alkylene group having 1 to 3 carbon atoms, and $R^3$ and $R^4$ represent an alkyl group having 1 to 3 carbon atom.) and/or an alkyl type quaternary ammonium represented by the general formula (II):

(Chemical formula 2)

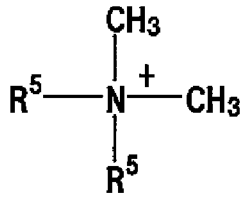

(II)

(wherein, $R^5$ represents a methyl group or a linear or branched hydrocarbon group having 11 to 25 carbon atoms, providing that at least one of two $R^5$s is a linear or branched hydrocarbon group having 11 to 25 carbon atoms.) is provided as an invention according to Claim 3.

A suitable range of the amino acid polymerization degree of a peptide portion of a peptide or peptide derivative having one or more acidic amino acid as the constituent amino acid varies depending on the kind of compounding cosmetic. The average degree of amino acid polymerization is preferably 2 to 500 in view of the stability of a cosmetic when compounded into the cosmetic and the ability of the cosmetic base material of the present invention for adsorbing onto hair. Thus, in the present invention, the cosmetic base material according to any one of Claims 1 to 3 wherein the average degree of amino acid polymerization of a peptide portion of the above-described peptide or peptide derivative is 2 to 500 is provided as an invention according to Claim 4.

As the peptide or peptide derivative having one or more acidic amino acid as the constituent amino acid, those which are industrially relatively easily available are preferable. Thus, in the present invention, the cosmetic base material according to any one of Claims 1 to 4 wherein the above-described peptide or peptide derivative is a protein hydrolyzate, a derivative of a protein hydrolyzate, a polyacidic amino acid or a derivative of a polyacidic amino acid is provided as an invention according to Claim 5.

An invention according to Claim 6 is the cosmetic base material according to any one of Claims 1 to 4 wherein the peptide portion of the peptide or peptide derivative having an acidic amino acid is a hydrolyzate of a plant protein or a keratin hydrolyzate.

The cosmetic base material of the present invention has an object of hydrophobizing the surface of hair of which its sensory characters lowered by losing the hydrophobicity of the hair surface by damages, thereby imparting flexibility, moisture retaining property, smoothness, luster and the like to hair. Therefore, it is preferable that two or more of surfactants are bonded to one molecule of the peptide or peptide derivative to form an ion complex. That is, it is preferable that per molecule of the peptide or peptide derivative, there exist a plurality of anionic functional groups to which surfactants as the component B can be bonded. In general, plant proteins and keratin contain acidic amino acids in an amount of 20 mol% or more and are industrially easily available. Thus, hydrolyzates thereof are suitable as the peptide portion of the peptide or peptide derivative to be used in producing the cosmetic base material of the present invention.

An invention according to Claim 7 is the cosmetic base material according to any one of Claims 1 to 4 wherein the peptide derivative is an acylated hydrolyzed protein, a glycerylated hydrolyzed protein, a quaternized hydrolyzed protein, a silylated hydrolyzed protein, an alkyl glycerylated hydrolyzed protein or a 2-hydroxyalkylated hydrolyzed protein.

The peptide derivative is preferably an acylated hydrolyzed protein, a glycerylated hydrolyzed protein, a quaternized hydrolyzed protein, a silylated hydrolyzed protein, an alkyl glycerylated hydrolyzed protein or a 2-hydroxyalkylated hydrolyzed protein, obtained by chemical modification of an amino group of a hydrolyzed protein, since these are industrially easily available and safe even if compounded in a cosmetic.

Since the cosmetic base material of the present invention has a characteristic that it is capable of hydrophobizing the surface of hair of which its sensory characters was lowered by losing its hydrophobicity by damages, thereby imparting flexibility, moisture retaining property, smoothness, luster and the like to hair, compounding thereof into a cosmetic is suitable.

Particularly when compounded in a hair conditioning agent such as a hair treatment and the like, the effect of the present invention is performed remarkably. In the present invention, a cosmetic comprising the cosmetic base material according to any one of Claims 1 to 7 is provided as an invention of Claim 8.

An invention according to Claim 9 is the cosmetic according to Claim 8 wherein the content of the above-described cosmetic base material is 0.1 to 30 % by mass. A suitable range of the content of the cosmetic base material of the present invention in a cosmetic varies depending on the kind of the cosmetic, use mode thereof and the like, and usually, the content is preferably 0.1 to 30 % by mass with respect to the total weight of the cosmetic.

Effect of the Invention

The cosmetic base material of the present invention shows high adsorption force onto hair and is capable of hydrophobizing the surface of hair having sensory characters lowered by losing the hydrophobicity of the hair surface by damages, thereby imparting flexibility, moisture retaining property, smoothness, luster and the like to hair. Therefore, the cosmetic base material is used suitably in a cosmetic, particularly, in a hair cosmetic, to manifest the above-described function. The cosmetic base material of the present invention can be compounded also in a skin cosmetic, to impart smoothness and moisture retaining property to dried skin.

Modes for carrying out the Invention

Next, modes for carrying out the present invention will be illustrated. First, the ion complex composed of anionic functional groups of a peptide or peptide derivative having one or more acidic amino acid as the constituent amino acid with at least one surfactant selected from the group consisting of cationic surfactants and ampholytic surfactants, constituting the characteristic part of the cosmetic base material of the present invention, is represented, for example, by the following general formula (III) and/or the following general formula (IV).

(Chemical formula 3)

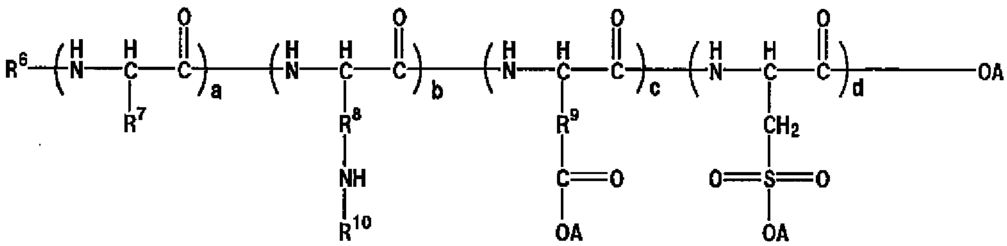

(III)

In the formula (III), $R^6$ and $R^{10}$ represent a hydrogen atom or a group represented by the general formula (VII), the general formula (VIII), the general formula (IX), the general formula (X), the general formula (XI) or the general formula (XIII) described later. $R^7$ represents a residue on the side chain of a neutral amino acid, $R^8$ represents a residue on a side chain excluding an amino group of a basic amino acid having an amino group on the side chain, and $R^9$ represents an alkylene group having 1 to 2 carbon atoms.

In the formula (III), "A" represents at least one surfactant (component B) selected from the group consisting of cationic surfactants and ampholytic surfactants, a hydrogen atom, an alkali metal, an alkaline earth metal, magnesium or the like. At least part of the moieties "A" are a surfactant as the component B, and preferably 30 mol% or more of the moieties "A" are surfactants as the component B.

Further, in the formula (III), a, b, c and d represent the number of each amino acid and a+b+c+d represents the amino acid polymerization degree, providing that a+b+c+d is 2 or more and c+d is 1 or more. Here, a, b, c and d represent only the number of amino acids and do not represent the order of an amino acid sequence. Though a, b, c, d, a+b+c+d, and c+d are theoretically integers, the peptide is often obtained as a mixture of those having different molecular weights, thus, these values are average values, and usually, these are represented by numbers other than integers in many cases.

(Chemical formula 4)

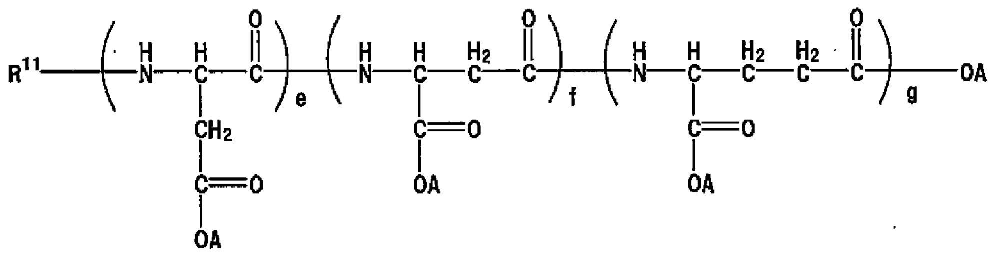

(IV)

In the formula (IV), $R^{11}$ represents a hydrogen atom or a group represented by the general formula (VII), the general formula (VIII), the general formula (IX), the general formula (X), the general formula (XI) or the general formula (XIII) described later.

In the formula (IV), "A" represents at least one surfactant (component B) selected from the group consisting of cationic surfactants and ampholytic surfactants, a hydrogen atom, an alkali metal, an alkaline earth metal, magnesium or the like. At least part of the moieties "A" are a surfactant as the component B, and preferably 30 mol% or more of the moieties "A" are surfactants as the component B.

Further, in the formula (IV), e, f and g represent the number of each amino acid and e+f+g represents the amino acid polymerization degree, providing that e+f+g is 2 or more. Here, e, f and g represent only the number of each amino acid, and do not represent the order of an amino acid sequence. Though e, f, g and e+f+g are theoretically integers, the peptide is often obtained as a mixture of those having different molecular weights, thus, these values are average values and usually, represented by numbers other than integers in many cases.

The average degree of amino acid polymerization of a peptide portion of the peptide or peptide derivative having one or more acidic amino acid as the constituent amino acid is preferably in the range of 2 to 500. Therefore, in the general formula (III), a+b+c is preferably 2 to 500, more preferably 3 to 400, further preferably 6 to 350. In the general formula (IV), e+f+g is preferably 2 to 500, more preferably 3 to 400, further preferably 6 to 350.

The preferable ranges of respective number of each amino acid unit constituting the above-described ion complex, that is, preferable ranges of respective numbers of basic amino acid units having an amino group on the side chain [amino acid unit appended with b in the formula (III)], acidic amino acid units having a carboxyl group on the side chain [amino acid unit appended with c in the formula (III) and with e, f or g in the formula (IV)], acidic amino acid units having a sulfo group on the side chain [amino acid unit appended with d in the formula (III)] and other amino acid units [amino acid unit appended with a in the formula (III)] in one molecule of the ion complex vary depending on use, raw material conditions, the kind of each amino acid unit and the like and are not particularly restricted. In the case of use in a usual cosmetic, a+b+c+d is in the range of 2 or more and 500 or less and a+b is preferably 0 to 485, more preferably 0 to 400, further preferably 3 to 300. In contrast, c+d is preferably 1 to 500, more preferably 1 to 300, further preferably 2 to 200.

In the formula (IV), e+f+g is in the range of 2 or more and 500 or less and e+f is preferably 0 to 500, more preferably 0 to 100, further preferably 0 to 30. g is preferably 0 to 500, more preferably 0 to 100, further preferably 0 to 30.

When a+b, c+d, e+f, and g are out of the above-described ranges, there is a possibility that sensory characters obtained by applying a hair treating agent and the like containing the cosmetic base material of the present invention on hair are deteriorated.

Next, the peptide, the peptide derivative, the surfactant as the component B and methods of forming the ion complex to be used in producing the cosmetic base material of the present invention will be illustrated in detail.

[Peptide]

The peptide portion of the peptide or peptide derivative to be used in constituting the cosmetic base material of the present invention contains an acidic amino acid including a carboxyl group and a sulfo group as the constituent amino acid, and
for example, is a peptide represented by the following general formula (V):

(Chemical formula 5)

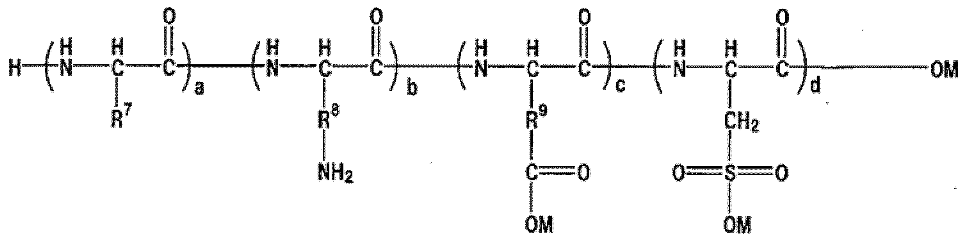

(V)

[in the formula (V), $R^7$ represents a residue on the side chain of a neutral amino acid, $R^8$ represents a residue on a side chain excluding an amino group of a basic amino acid having an amino group on the side chain, and $R^9$ represents an alkylene group having 1 to 2 carbon atoms. M represents a hydrogen atom, an alkali metal, an alkaline earth metal and/or magnesium. Further, a, b, c and d represent the number of each amino acid and a+b+c+d represents the amino acid polymerization degree, providing that a+b+c+d is 2 or more and c+d is 1 or more. Here, a, b, c and d represent only the number of each amino acid and do not represent the order of an amino acid sequence. Though a, b, c, d, a+b+c+d, and c+d are theoretically integers, the peptide is often obtained as a mixture of those having different molecular weights, thus, these values are average values and usually, represented by numbers other than integers in many cases.], or

a polyacidic amino acid, such as polyaspartic acid, polyglutamic acid [poly(γ-glutamic acid)] and the like, represented by the following general formula (VI):

(Chemical formula 6)

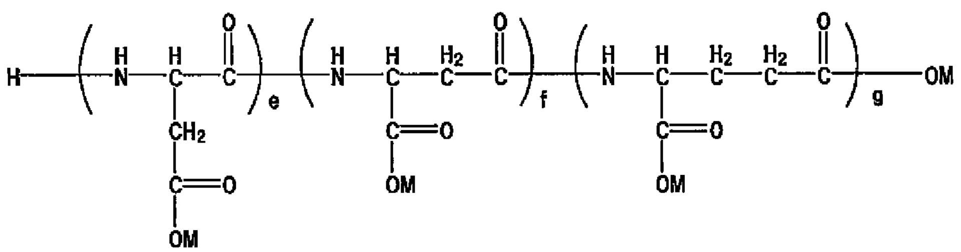

(VI)

[in the formula (VI), M represents a hydrogen atom, an alkali metal, an alkaline earth metal and/or magnesium. Further, e, f and g represent the number of each amino acid and e+f+g represents the amino acid polymerization degree, providing that e+f+g is 2 or more. Here, e, f and g represent only the number of each amino acid and do not represent the order of an amino acid sequence. Though e, f, g and e+f+g are theoretically integers, the peptide is often obtained as a mixture of those having different molecular weights, thus, these values are average values and usually, represented by numbers other than integers in many cases.]. It is preferable to use protein hydrolyzates (also called hydrolyzed protein) obtained by hydrolyzing proteins since these are industrially available and from the standpoint of safeness in compounding the cosmetic base material of the present invention into a cosmetic.

The above-described protein hydrolyzates are obtained by partial hydrolysis of proteins with an acid, an alkali, an enzyme or a combination thereof. Protein sources thereof include animal proteins, plant proteins, microorganism-derived proteins and the like. Examples of the animal protein include collagen (including also gelatin as a modified substance thereof), keratin, fibroin, sericin, casein, conchiolin, elastin, protamine, egg yolk proteins and egg albumen proteins of chicken and the like, etc. Examples of the plant protein include proteins contained in soybean, wheat, rice (rice bran), sesame, pea, corn, potatoes and the like. Examples of the microorganism-derived protein include yeast proteins separated from Saccharomyces, Candida and Endomycopsis yeasts and yeasts called beer yeast and Sake yeast, proteins separated from fungi (basidiomycete) and chlorella, spirulina proteins derived from sea algae, and the like.

In the cosmetic base material of the present invention, it is necessary that at least some of anionic functional groups of a peptide or peptide derivative form an ion complex with a cationic surfactant and/or an ampholytic surfactant. Thus, it is required that a peptide portion of the peptide or peptide derivative contains an acidic amino acid as the constituent amino acid. Among the above-described proteins, plant proteins and keratin and casein are suitable as the protein source for forming an ion complex in the cosmetic base material of the present invention since a large amount

of acidic amino acids are contained therein, and plant protein hydrolyzates, keratin hydrolyzates (hydrolyzed keratin) and casein hydrolyzates (hydrolyzed casein) obtained by hydrolyzing these proteins are suitable as the peptide portion of a peptide or peptide derivative to be used in constituting the cosmetic base material of the present invention. Among them, especially, plant protein hydrolyzates are preferable.

Further, also polyacidic amino acids such as polyaspartic acid, polyglutamic acid [poly(γ-glutamic acid)] and the like can be used as the peptide portion of a peptide or peptide derivative having an acidic amino acid as the constituent amino acid to be used in constituting the cosmetic base material of the present invention.

The peptides which are suitable for use in constituting the cosmetic base material of the present invention are discussed as described above, and plant protein hydrolyzates, keratin hydrolyzates, casein hydrolyzates, polyaspartic acid and poly(γ-glutamic acid) are preferable, and among them, especially, plant protein hydrolyzates are preferable, as the peptide portion of peptide or peptide derivative, in view of sensory characters and an ability of adsorbing onto hair in applying the cosmetic base material of the present invention to hair, in addition to industrial easy availability and high safeness when compounded into a cosmetic.

[Peptide derivative]

The peptide derivative having one or more acidic amino acid as the constituent amino acid as the component A constituting the cosmetic base material of the present invention corresponds to a peptide derivative having peptide amino groups modified chemically. That is, the peptide derivative is a peptide derivative in which at least some hydrogen atoms of terminal amino groups of the peptide main chain and amino groups of the amino acid side chain in the above-described general formula (V) are substituted by functional groups or a peptide derivative in which at least some hydrogen atoms of terminal amino groups of the peptide main chain in the above-described general formula (VI) are substituted by functional groups. Specific examples thereof include acylated peptide, glycerylated peptide, quaternized peptide, silylated peptide, alkyl glycerylated peptide, 2-hydroxyalkylated peptide and the like.

The above-described acylated peptide corresponds to those in which a linear or branched saturated or unsaturated fatty acid or resin acid having 8-32 carbon atoms is bonded to terminal amino groups of the peptide main chain and amino groups of the amino acid side chain to form an amide bond. That is, the acylated peptide corresponds to those in which a residue excluding a -OH group in a carboxyl group of a linear or branched saturated or unsaturated fatty acid or resin acid having 8-32 carbon atoms is bonded to at least some of terminal amino groups of the peptide main chain and amino groups of the amino acid side chain in the above-described general formula (V) or at least some of terminal amino groups of the peptide main chain in the above-described general formula (VI).

Examples of the above-described linear or branched saturated or unsaturated fatty acid or resin acid having 8-32 carbon atoms include lauric acid, myristic acid, coco-fatty acid, isostearic acid, stearic acid, undecylenic acid, lanolin fatty acid, resin acid, hydrogenated resin acid and the like. Examples of the acylated peptide salt include potassium salts, sodium salts, triethanolamine salt, 2-amino-2-methyl-1,3-propanediol salt, 2-amino-2-methyl-1-propanol salt and the like.

Since protein hydrolyzates (also called hydrolyzed protein) are industrially easily available and show high safeness for human bodies as described above, it is preferable to use a protein hydrolyzate also in the peptide portion of the acylated peptide, and an acylated hydrolyzed protein is preferable as the acylated peptide to be used in constituting the cosmetic base material of the present invention.

The glycerylated peptides include those in which a group represented by the following general formula (VII):

(Chemical formula 7)

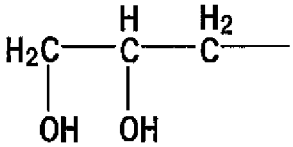

(VII)

or a group represented by the following general formula (VIII):

(Chemical formula 8)

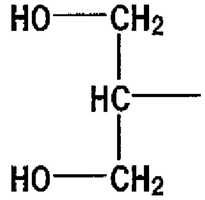

(VIII)

is bonded to at least some of terminal amino groups of the peptide main chain and amino groups of the amino acid side chain represented in the above-described general formula (V) or at least some of terminal amino groups of the peptide main chain represented in the above-described general formula (VI). This glycerylated peptide can be produced, for example, by a method described in JP-2005-306799A.

Similar to the case of the above-described acylated peptide, it is preferable to use a protein hydrolyzate also in the peptide portion of the glycerylated peptide, and a glycerylated hydrolyzed protein is preferable as the glycerylated peptide to be used in constituting the cosmetic base material of the present invention.

The quaternized peptides include those in which a group represented by the following general formula (IX):

(Chemical formula 9)

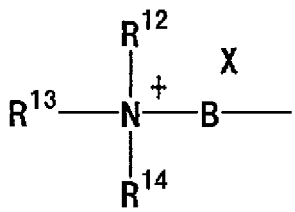

(IX)

[in the formula (IX), $R^{12}$, $R^{13}$ and $R^{14}$ may be the same or different and represent an alkyl group having 1 to 22 carbon atoms or an alkenyl group having 2 to 22 carbon atoms, alternatively, one or two of $R^{12}$ to $R^{14}$ represent an alkyl group having 1 to 22 carbon atoms or an alkenyl group having 2 to 22 carbon atoms, the residual group represents an alkyl group having 1 to 3 carbon atoms, a hydroxyalkyl group having 1 to 3 carbon atoms or a benzyl group. B represents a saturated hydrocarbon having 2 to 3 carbon atoms or a saturated hydrocarbon having 2 to 3 carbon atoms and substituted with a hydroxyl group, and X represents a halogen atom] is bonded to at least some of terminal amino groups of the peptide main chain and amino groups of the amino acid side chain in the above-described general formula (V) or at least some of terminal amino groups of the peptide main chain in the above-described general formula (VI). The quaternized peptide can be obtained by reacting a peptide and a quaternary ammonium compound under alkali conditions.

Specific examples of the above-described quaternary ammonium compound include glycidyl ammonium salt such as glycidylstearyldimethyl ammonium chloride, glycidylcocoalkylatedimethyl ammonium chloride, glycidyllauryldimethyl ammonium chloride, glycidyltrimethyl ammonium chloride and the like; 3-halo-2-hydroxypropyl ammonium salt such as 3-chloro-2-hydroxypropyl stearyldimethyl ammonium chloride, 3-chloro-2-hydroxypropyl cocoalkylatedimethyl ammonium chloride, 3-chloro-2-hydroxypropyllauryldimethyl ammonium chloride, 3-chloro-2-hydroxypropylethyldimethyl ammonium chloride, 3-chloro-2-hydroxypropyltrimethyl ammonium chloride and the like; 2-halo-ethyl ammonium salt such as 2-chloroethyltrimethyl ammonium chloride and the like; and 3-halo-propyl ammonium salt such as 3-chloropropyltrimethyl ammonium chloride.

Similar to the case of the above-described acylated peptide, a protein hydrolyzate is preferable in the peptide portion of the quaternized peptide, and a quaternized hydrolyzed protein is preferable as the quaternized peptide to be used in constituting the cosmetic base material of the present invention.

The silylated peptide includes to those in which a group represented by the following general formula (X):

(Chemical formula 10)

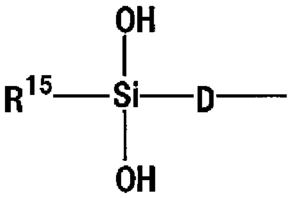

(X)

[in the formula (X), $R^{15}$ represents an alkyl having 1 to 3 carbon atoms, D is a connecting bond and represents methylene, propylene, or a group represented by $-CH_2OCH_2CH(OH)CH_2-$ or $-(CH_2)_3OCH_2CH(OH)CH_2-$] is bonded to at least some of terminal amino groups of the peptide main chain and amino groups of the amino acid side chain in the above-described general formula (V) or at least some of terminal amino groups of the peptide main chain in the above-described general formula (VI). This silylated peptide can be produced, for example, by methods described in JP-8-059424A, JP-8-067608A and the like.

Silylated peptide easily causes mutual dehydration condensation of silanol groups to form a siloxane bond, by heating, giving a polymer (hereinafter, this polymer is referred to as "silicone resinated peptide"). Therefore, the silylated peptide used in the present invention includes those containing a silicone resinated peptide which is a polymer of a peptide obtained by bonding a group of the general formula (X) to an amino group, in addition to a peptide monomer obtained by bonding a group of the general formula (X) to an amino group.

Similar to the case of the above-described acylated peptide, it is preferable to use a protein hydrolyzate also in the peptide portion of the silylated peptide, and a silylated hydrolyzed protein is preferable as the silylated peptide to be used in constituting the cosmetic base material of the present invention.

The alkyl glycerylated peptide includes those in which a group represented by the following general formula (XI):

(Chemical formula 11)

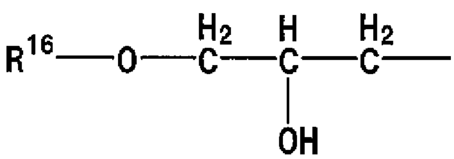

(XI)

[in the formula (XI), $R^{16}$ represents an alkyl group having 1 to 22 carbon atoms, an alkenyl group having 2 to 22 carbon atoms or a phenyl group] is bonded to at least some of terminal amino groups of the peptide main chain and amino groups of the amino acid side chain in the above-described general formula (V) or at least some of terminal amino groups of the peptide main chain in the above-described general formula (VI). This alkyl glycerylated peptide is obtained by reacting a peptide and an alkyl glycerylating agent represented by the following general formula (XII):

(Chemical formula 12)

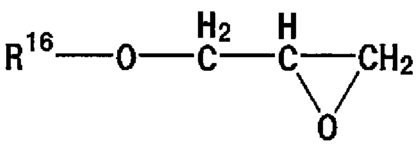

(XII)

[in the formula (XII), $R^{16}$ represents an alkyl group having 1 to 22 carbon atoms, an alkenyl group having 2 to 22 carbon atoms or a phenyl group] under alkali conditions.

The temperature for reacting a peptide and an alkyl glycerylating agent is preferably 30°C to 80°C, more preferably 40°C to 70°C. Below the above-described temperature, reactivity of the peptide and the alkyl glycerylating agent deteriorates. Over the above-described temperature, the peptide generates remarkable coloration and unpleasant

odor, not suitable for a cosmetic base material, and improvement in reactivity by raising the reaction temperature is not expected.

Although the reaction time in reacting a peptide and an alkyl glycerylating agent varies depending on the temperature, pH and peptide concentration, it is preferably 1 to 10 hours, more preferably 2 to 8 hours. When the reaction time is shorter than the above-described time, the unreacted alkyl glycerylating agent tends to remain, and when the reaction time is longer than the above-described time, the peptide tends to generate coloration and unpleasant order.

Similar to the case of the above-described acylated peptide, it is preferable to use a protein hydrolyzate also in the peptide portion of the alkyl glycerylated peptide, and an alkyl glycerylated hydrolyzed protein is preferable as the alkyl glycerylated peptide to be used in constituting the cosmetic base material of the present invention.

The 2-hydroxyalkylated peptide includes those in which a group represented by the following general formula (XIII):

(Chemical formula 13)

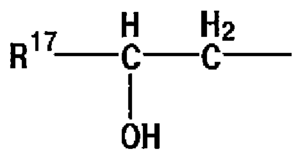

(XIII)

[in the formula (XIII), $R^{17}$ represents an alkyl group having 1 to 20 carbon atoms, an alkenyl group having 2 to 20 carbon atoms or a phenyl group] is bonded to at least some of terminal amino groups of the peptide main chain and amino groups of the amino acid side chain in the above-described general formula (V) or at least some of terminal amino groups of the peptide main chain in the above-described general formula (VI). This 2-hydroxyalkylated peptide is obtained by heating and reacting a peptide and a 2-hydroxyalkylating agent represented by the following general formula (XIV):

(Chemical formula 14)

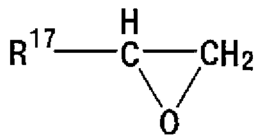

(XIV)

[in the formula (XIV), $R^{17}$ represents an alkyl group having 1 to 20 carbon atoms, an alkenyl group having 2 to 20 carbon atoms or a phenyl group] under alkali conditions.

The temperature in reacting a peptide and a 2-hydroxyalkylating agent is preferably 30°C to 80°C, more preferably 40°C to 70°C. Below the above-described temperature, reactivity of the peptide and the 2-hydroxyalkylating agent tends to deteriorate, and over the above-described temperature, the peptide generates remarkable coloration and unpleasant odor, not suitable for a cosmetic base material, and improvement in reactivity by raising the reaction temperature is not expected.

Although the reaction time in reacting a peptide and a 2-hydroxyalkylating agent varies depending on the temperature, pH and peptide concentration, it is preferably 1 to 10 hours, more preferably 2 to 8 hours. When the reaction time is shorter than the above-described time, the unreacted 2-hydroxyalkylating agent tends to remain, and when the reaction time is longer than the above-described time, the peptide tends to generate coloration and unpleasant odor.

Similar to the case of the above-described acylated peptide, it is preferable to use a protein hydrolyzate also in the peptide portion of the 2-hydroxyalkylated peptide, and a 2-hydroxyalkylated hydrolyzed protein is preferable as the 2-hydroxyalkylated peptide to be used in constituting the cosmetic base material of the present invention.

[Surfactant as component B]

The surfactant (component B) forming an ion complex by ionically bonding to anionic functional groups of a peptide or peptide derivative (component A) having one or more acidic amino acid as the constituent amino acid is a cationic surfactant or an ampholytic surfactant.

Examples of the cationic surfactant include amine salt type cationic surfactants such as fatty acid amide amine

salts, alkylamine salts, alkylaminepolyoxyethylene adducts, fatty acid triethanolamine monoester salts, fatty acid polyamine condensates and the like, quaternary ammonium salt type cationic surfactants such as alkyl type quaternary ammonium salts including monoalkyl trimethyl ammonium salts and dialkyl dimethyl ammonium salts, benzalkonium type quaternary ammonium salts, alkyl pyridinium salts, acylamino alkyl type ammonium salts, acylamino alkyl pyridinium salts, diacyloxyethylammonium salts and the like, and imidazoline and imidazolium salt type cationic surfactants.

Examples of the above-described ampholytic surfactant include acylamino acid type ampholytic surfactants obtained by acylation of basic amino acids such as arginine, lysine, histidine, ornithine and the like, betaine type ampholytic surfactants such as alkyl dimethyl aminoacetic acid betaine, alkylamide propyl dimethyl ammonio acetate, alkyl dimethyl aminosulfo betaine and the like, imidazoline type ampholytic surfactants, acylated derivatives of protein hydrolyzates obtained by hydrolyzing proteins containing a large amount of basic amino acids such as protamine and the like; etc.

Among the above-described surfactants, fatty acid amide amine salts, alkyl type quaternary ammoniums such as monoalkyl trimethyl ammonium salts, dialkyl dimethyl ammonium salts and the like, alkyl amine salts, acyl amino acids obtained by acylating basic amino acids, and the like are preferable.

That is, fatty acid amide amine salts, alkyl type quaternary ammoniums such as monoalkyl trimethyl ammonium salts, dialkyl dimethyl ammonium salts and the like, alkyl amine salts, acyl amino acids obtained by acylating basic amino acids, and the like are listed as preferable surfactants since these substances easily form an ion complex with anionic functional groups of a peptide or peptide derivative (component A) having one or more acidic amino acid as the constituent amino acid and these substances can be produced at industrially with relatively lower cost. Among them, particularly, fatty acid amide amine salts and alkyl type quaternary ammoniums such as monoalkyl trimethyl ammonium salts, dialkyl dimethyl ammonium salts and the like are preferable. Among these fatty acid amide amine salts, fatty acid amide amines represented by the above-described general formula (I) are mentioned as substances of high utility, and among the alkyl type quaternary ammoniums, alkyl type quaternary ammoniums represented by the above-described general formula (II) are mentioned as substances of high utility.

[Method for forming ion complex]

The ion complex in the cosmetic base material of the present invention is composed of an ion complex of at least some of anionic functional groups of a peptide or peptide derivative (component A) having one or more acidic amino acid as the constituent amino acid with at least one surfactant (component B) selected from the group consisting of cationic surfactants and ampholytic surfactants, and is formed by ionic bonding of anionic functional groups of a peptide or peptide derivative as the component (A) and a surfactant as the component (B) described previously.

As the ion complex in the cosmetic base material of the present invention, ion complexes formed by ionic bond of 30 mol% or more of all anionic functional groups of a peptide or peptide derivative having one or more acidic amino acid as the constituent amino acid with a fatty acid amide amine represented by the general formula (I) and/or an alkyl type quaternary ammonium represented by the general formula (II) are particularly preferable, since the ion complexes are easily produced industrially. Ion complexes obtained by using fatty acid amide amines are particularly preferable because of excellent sensory characters in applying to hair.

As a method for forming the ion complex in the cosmetic base material of the present invention,for example, following method can be mentioned. A peptide or peptide derivative is converted into a solution with a solvent such as water, lower alcohols including ethanol, isopropanol and the like, polyhydric alcohols such as propylene glycol, butylene glycol, glycerin and the like, and, then, an acid agent such as hydrochloric acid, sulfuric acid and the like is added to the peptide or peptide derivative solution to make the peptide or peptide derivative solution weak acidic to acidic conditions having a pH of 5 or less, preferably 3 or less, more preferably 2.5 or less. Subsequently, this weak acidic to acidic peptide or peptide derivative solution is subjected to an electrodialyzer or/and a dialysis tube, to perform a desalting treatment. If the peptide or peptide derivative used is insolubilized under weak acidic to acidic conditions, a de-salted peptide or peptide derivative can be obtained by carrying out filtration and decantation to recover the residue.

Next, an ion complex can be formed by adding a surfactant as the component B to the de-salted peptide or peptide derivative solution. The cosmetic base material of the present invention having higher purify can be obtained if, in the step of adding surfactant, an ion complex is formed by neutralizing the de-salted weak acidic to acidic peptide or peptide derivative solution by adding a basic surfactant such as a fatty acid amide amine represented by the general formula (I).

Since a surfactant having a strong cationic functional group such as acylarginine and an alkyl type quaternary ammonium represented by the general formula (II) easily forms an ion complex with a peptide or a peptide derivative, the step of adding an acid agent to adjust pH to 5 or less and carrying out a de-salting treatment using an electrodialyzer and a dialysis tube as described above can be omitted, and an ion complex can be produced by adjusting pH of the peptide or peptide derivative solution to 5 to 8, then, adding an alkyl type quaternary ammonium or acylarginine.

Further, in the case of a basic surfactant such as a fatty acid amide amine represented by the general formula (I), an ion complex can be formed by converting the fatty acid amide amine into a fatty acid amide amine salt with

hydrochloric acid or sulfuric acid, and adding the salt to a solution of a peptide or peptide derivative. In this case, since the fatty acid amide amine salt easily forms an ion complex with a peptide or peptide derivative, the pretreatment of adding an acid agent to a solution of a peptide or peptide derivative to adjust pH to 5 or lower before addition of the fatty acid amide amine salt and carrying out a de-salting treatment using an electrodialyzer or/and a dialysis tube can be omitted.

The ion complexes formed as described above will be exemplified below. Formation of an in complex of a peptide or peptide derivative with a surfactant occurs at anionic functional groups of a peptide or peptide derivative, that is, at a carboxyl group and a sulfo group. Hence, a case of generation of an ion complex at a carboxyl group and a case of generation of an ion complex at a sulfo group will be explained separately.

In the case of formation of an ion complex of a peptide or peptide derivative with a fatty acid amide amine represented by the general formula (I) at a carboxyl group of the peptide or peptide derivative, the ion complex is represented by the following general formula (XV). The peptide or peptide derivative is represented by the general formula (III) as described above, and the position of formation of an ion complex by the peptide or peptide derivative with a fatty acid amide amine is a carboxyl group in this example. For simplification, only the carboxyl group part is extracted and denoted and other parts are represented by "peptide", in the peptide or peptide derivative. If the peptide or peptide derivative is wholly displayed, a detailed and wider area is occupied as in the general formula (III), so only a characteristic part of formation of an ion complex is simply displayed.

(Chemical formula 15)

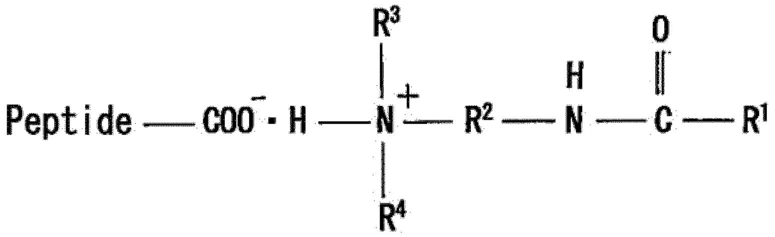

(XV)

In the case of formation of an ion complex by an alkyl type quaternary ammonium represented by the general formula (II) at a carboxyl group of a peptide or peptide derivative, the ion complex is represented by the following general formula (XVI). The method of displaying the peptide or peptide derivative is the same as described above.

(Chemical formula 16)

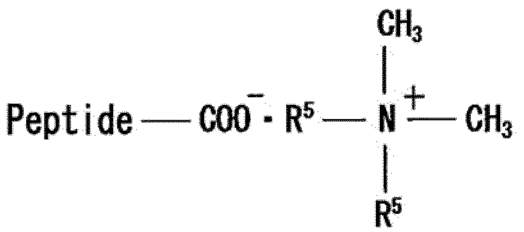

(XVI)

Next, in the case of formation of an ion complex by a fatty acid amide represented by the general formula (I) at a sulfo group of a peptide or peptide derivative, the ion complex is represented by the following general formula (XVII). Only the sulfo group part is extracted and denoted and other parts are represented by "peptide", in the peptide or peptide derivative, like the above-described case of a carboxyl group.

(Chemical formula 17)

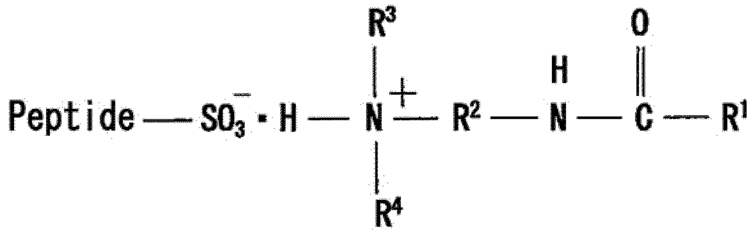

(XVII)

In the case of formation of an ion complex by an alkyl type quaternary ammonium represented by the general formula (II) at a sulfo group of a peptide or peptide derivative, the ion complex is represented by the following general formula (XVIII). The display method for the peptide or peptide derivative is the same as described above.

(Chemical formula 18)

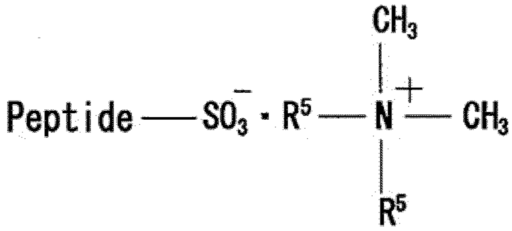

(XVIII)

In the present invention, the proportion of anionic functional groups of a peptide or peptide derivative forming an ion complex with a surfactant as the component B is preferably 30 mol% or more with respect to all anionic functional groups, since if the proportion of anionic functional groups forming an ion complex is 30 mol% or more with respect to all anionic functional groups, the effect of the ion complex of imparting flexibility, moisture retaining property, smoothness, luster and the like to hair is appropriately manifested. For more suitable manifestation of the properties described above, a larger number of anionic functional groups forming the ion complex is more preferable. From this standpoint, the proportion of anionic functional groups forming the ion complex is more preferably 50 mol% or more with respect to all anionic functional groups. The proportion of anionic functional groups forming the ion complex may also be 100 mol% with respect to all anionic functional groups (namely, all anionic functional groups form the ion complex).

The peptide or peptide derivative having the ion complex formed as described above can be used as it is in the cosmetic base material of the present invention. If it is in the form of solution after formation of the ion complex, the solution can be further subjected to a de-salting treatment using an electrodialyzer or/and a dialysis tube and can be used in more purified condition in the cosmetic base material of the present invention.

When the ion complex formed by adding a surfactant as the component B to a peptide or peptide derivative in the form of solution is an insoluble substance, the insolubilized ion complex can be washed with a suitable solvent such as water, lower alcohol containing water and the like and used in more highly purified condition in the cosmetic base material of the present invention, though the peptide or peptide derivative having the ion complex can be used as it is in the cosmetic base material of the present invention.

For obtaining an ion complex of a peptide derivative with a surfactant as the component B, it is preferable that a peptide is derivatized as described above, then, allowed to form an ion complex with a surfactant as the component B. In the case of use of a silylated peptide, however, if there is a situation of avoiding as much as possible mutual polymerization of silylated peptides during formation of an ion complex with a surfactant as the component B, it may be permissible that after formation of an ion complex of a peptide with a surfactant as the component B, a functional group represented by the above-described general formula (X) is bonded to an amino group of the constituent peptide of the ion complex. However, in this case, if the ion complex formed previously is insoluble, an amino group of the peptide cannot be derivatized easily, thus, it is preferable that the ion complex is dispersed appropriately using a lower alcohol, a poly-hydric alcohol or the like to prepare a solution, then, it is derivatized.

Next, applications of the cosmetic base material of the present invention, that is, a cosmetic base material composed of a peptide or peptide derivative in which at least some of anionic functional groups of a peptide or peptide derivative (component A) having one or more acidic amino acid as the constituent amino acid form an ion complex with

at least one surfactant (component B) selected from the group consisting of cationic surfactants and ampholytic surfactants will be explained.

Examples of the cosmetic into which the cosmetic base material of the present invention is compounded include hair cosmetics such as shampoo, hair rinse, hair conditioner, split hair coating lotion, hair cream, permanent wave first agent (reducing agent) and second agent (oxidizing agent), hair set lotion, hair dye, hair coloring preparation, liquid hair styling preparation, hair tonic, hair growth tonic and the like; skin cosmetics such as cleansing cream, emollient cream, hand cream, after shaving lotion, shaving foam, facial cleansing cream, facial wash, body shampoo, various soaps, depilatory, face pack, milky lotion, skin lotion, makeup article, sunscreen article and the like.

A preferable range of the compounding amount of the cosmetic base material of the present invention (content in a cosmetic) varies depending on the kind of a cosmetic and is not particularly restricted. It is preferably 0.1 to 30 % by mass in the cosmetic, and particularly, preferably about 1 to 20 % by mass in the cosmetic in many cases. When the compounding amount into the cosmetic is smaller than the above-described range, there is a possibility that the effect of imparting excellent flexibility, moisture retaining property, smoothness and luster to hair and skin is not sufficiently manifested. Even if the compounding amount of the cosmetic base material of the present invention is over the above-described range, there is no improvement in the effect corresponding to the increase in the amount.

Examples of components which can be compounded together with the cosmetic base material of the present invention into the above-described cosmetic include anionic surfactants, nonionic surfactants, ampholytic surfactants, cationic surfactants, synthetic polymers such as cationic polymers, ampholytic polymers, anionic polymers and the like, semisynthetic polymers, animal and vegetable oils, hydrocarbons, ester oils, higher alcohols, amino acids, thickening agents, animal and plant extracts, silicones, preservatives, perfumes, protein hydrolyzates obtained by hydrolyzing animal and plant-derived and microorganism-derived proteins, and esterified derivatives of these protein hydrolyzates, quaternary ammonium derivatives, silylated derivatives, acylated derivatives and salts thereof, and the like. Other components can be appropriately added as long as not deteriorating the property of the cosmetic base material of the present invention.

## Examples

Next, the present invention will be illustrated specifically with examples, but the present invention is not limited only to these examples. In examples, % is % by mass in all cases. Before explanation of the examples, a method of measuring the amino nitrogen and the total nitrogen amount and a method of estimating the mole number of all anionic functional groups adopted in examples will be explained.

### [Method of measuring amino nitrogen and total nitrogen amount]

In examples, measurement of the amino nitrogen amount was carried out according to the van Slyke method. Measurement of the total nitrogen amount was carried out according to the improved Dumas method.

### [Method of estimating mole number of all anionic functional groups]

The mole number of all anionic functional groups in a peptide or peptide derivative having one or more acidic amino acid as the constituent amino acid was measured as described below. That is, it was hypothesized that terminal carboxyl groups were present in equimolar amount to the mole number of amino nitrogen calculated from the amino nitrogen amount measured by the van Slyke method, and the mole number of acidic amino acids (glutamic acid, aspartic acid and cysteic acid) measured by amino acid analysis was considered as the total mole number of side chain carboxyl groups and sulfo groups of the peptide or peptide derivative. The sum of the mole number of terminal carboxyl groups and the mole number of side chain carboxyl groups and sulfo groups was estimated as the mole number of all anionic functional groups in the peptide or peptide derivative.

The amino acid polymerization degree of a peptide portion in a peptide or peptide derivative used in examples [namely, a+b+c+d in the general formula (V) or e+f+g in the general formula (VI)] was calculated based on the proportion of the total nitrogen amount to the amino nitrogen amount. Further, a:b:c:d and e+f:g were obtained by amino acid analysis using an amino acid automatic analyzer manufactured by Hitachi Ltd., and based on the amino acid polymerization degree, values of a, b, c, d, e+f and g were obtained. Protein hydrolyzates used in examples were obtained by hydrolyzing respective protein sources by a known method using an acid, an alkali or an enzyme.

Example 1:

[Formation of ion complex of wheat protein hydrolyzate with N-[2-(diethylamino)ethyl]octadecaneamide (in the general formula (I), $R^1$ is a heptadecyl group, $R^2$ is an ethylene group, $R^3$ is an ethyl group and $R^4$ is an ethyl group. Hereinafter, this is called "diethylaminoethylstearamide")]

To 400 g of a 30% aqueous solution of a wheat protein hydrolyzate [in the general formula (V), a is 3.0, b is 0.1, c is 2.4, d is 0.2 and a+b+c+d is 5.7], dilute hydrochloric acid was added to adjust pH to 2.7. This wheat protein hydrolyzate aqueous solution was de-salted using an electrodialyzer, to obtain an aqueous solution of a wheat protein hydrolyzate containing anionic functional groups in free condition (-COOH or $SO_3H$).
Next, 400 g of the above-described wheat protein hydrolyzate aqueous solution of which concentration had been adjusted to 25% (the mole number of all anionic functional groups was 0.44 mol) was stirred with heating at 70°C, and, into this, diethylaminoethylstearamide (84 g, 50 mol% with respect to all anionic functional groups in the wheat protein hydrolyzate) was added, and the mixture was stirred with heating at 70°C until homogeneous.
By the above-described treatment, diethylaminoethylstearamide was ionically bonded to 50 mol% of anionic functional groups in all anionic functional groups of the wheat protein hydrolyzate to form an ion complex. Thus, 484 g of an aqueous solution of the wheat protein hydrolyzate was obtained, which has an ion complex with diethylaminoethylstearamide and has a solid concentration of 38%.

Example 2

[Formation of ion complex of wheat protein hydrolyzate with diethylaminoethylstearamide, N-[2-(diethylamino)ethyl] docosaneamide (in the general formula (I), $R^1$ is a heneicosyl group, $R^2$ is an ethylene group, $R^3$ is an ethyl group and $R^4$ is an ethyl group. Hereinafter, this is called "diethylaminoethylbehenamide"), and N-[2-(diethylamino)ethyl]iso-octadecaneamide (in the general formula (I), $R^1$ is an iso-heptadecyl group, $R^2$ is an ethylene group, $R^3$ is an ethyl group and $R^4$ is an ethyl group. Hereinafter, this is called "diethylaminoethylisostearamide")]

According to the same method as the method in Example 1, the same wheat protein hydrolyzate as that used in Example 1 was processed to obtain an aqueous solution of a wheat protein hydrolyzate containing anionic functional groups in free condition (-COOH or $SO_3H$).
Next, 400 g of the above-described wheat protein hydrolyzate aqueous solution of which concentration had been adjusted to 25% (the mole number of all anionic functional groups was 0.44 mol) was stirred with heating at 80°C, and, into this, diethylaminoethylstearamide (75.9 g), diethylaminoethylbehenamide (8.0 g) and diethylaminoethylisostearamide (0.1 g) (Total amount of the three fatty acid amide amine is 50 mol% with respect to all anionic functional groups in the wheat protein hydrolyzate.)were added, and the mixture was stirred with heating at 80°C until homogeneous.
By the above-described treatment, diethylaminoethylstearamide, diethylaminoethylbehenamide and diethylaminoethylisostearamide were ionically bonded to 50 mol% of anionic functional groups in all anionic functional groups of the wheat protein hydrolyzate to form an ion complex. Thus, 484 g of an aqueous solution of the wheat protein hydrolyzate was obtained, which has an ion complex with diethylaminoethylstearamide, diethylaminoethylbehenamide and diethylaminoethylisostearamide, and has solid concentration of 38%.

Example 3

[Formation of ion complex of wheat protein hydrolyzate with diethylaminoethylstearamide, diethylaminoethylbehenamide and diethylaminoethylisostearamide]

According to the same method as the method in Example 1, the same wheat protein hydrolyzate as that used in Example 1 was processed to obtain an aqueous solution of a wheat protein hydrolyzate containing anionic functional groups in free condition (-COOH or $SO_3H$).
Next, 400 g of the above-described wheat protein hydrolyzate aqueous solution of which concentration had been adjusted to 25% (the mole number of all anionic functional groups was 0.44 mol) was stirred with heating at 80°C, and, into this, diethylaminoethylstearamide (34 g), diethylaminoethylbehenamide (20 g) and diethylaminoethylisostearamide (30 g) (Total amount of the three fatty acid amide amine is 50 mol% with respect to all anionic functional groups in the wheat protein hydrolyzate.)were added, and the mixture was stirred with heating at 80°C until homogeneous.
By the above-described treatment, diethylaminoethylstearamide, diethylaminoethylbehenamide and diethylaminoethylisostearamide were ionically bonded to 50 mol% of anionic functional groups in all anionic functional groups of the wheat protein hydrolyzate to form an ion complex. Thus, 484 g of an aqueous solution of the wheat protein hydrolyzate was obtained, which has an ion complex with diethylaminoethylstearamide, diethylaminoethylbehenamide

and diethylaminoethylisostearamide, and has solid concentration of 38%.

Example 4

[Formation of ion complex of pea protein hydrolyzate with diethylaminoethylstearamide, diethylaminoethylbehenamide and diethylaminoethylisostearamide]

To 400 g of a 25% aqueous solution of a pea protein hydrolyzate [in the general formula (V), a is 55.3, b is 10.5, c is 32.9, d is 1.3 and a+b+c+d is 100] (the mole number of all anionic functional groups was 0.29 mol), dilute hydrochloric acid was added to adjust pH to 2.5, and the pea protein hydrolyzate was insolubilized and precipitated. To the insolubilized pea protein hydrolyzate, 1000 ml of water was added and stirred, then removing the water layer by decantation. Thereafter, salts contained in the pea protein hydrolyzate was de-salted to obtain a pea protein hydrolyzate containing anionic functional groups in free condition (-COOH or $SO_3H$).

Next, the above-described de-salted pea protein hydrolyzate solution was stirred with heating at 80°C, and, into this, diethylaminoethylstearamide (39.7 g), diethylaminoethylbehenamide (19.9 g) and diethylaminoethylisostearamide (6.6 g) (Total amount of the three fatty acid amide amine is 50 mol% with respect to all anionic functional groups in the pea protein hydrolyzate.)were added, and the mixture was stirred with heating at 80°C until homogeneous.

By the above-described treatment, diethylaminoethylstearamide, diethylaminoethylbehenamide and diethylaminoethylisostearamide were ionically bonded to 50 mol% of anionic functional groups in all anionic functional groups of the pea protein hydrolyzate to form an ion complex, to which water was added to adjust solid concentration to 40%. Thus, 341 g of an aqueous solution of the pea protein hydrolyzate was obtained, which has an ion complex with diethylaminoethylstearamide, diethylaminoethylbehenamide and diethylaminoethylisostearamide, and has solid concentration of 40%.

Example 5

[Formation of ion complex of keratin hydrolyzate with stearyl trimethylammonium(in the general formula (II), one of $R^5$ is a stearyl group and other one is a methyl group)]

A 25% aqueous solution of a keratin hydrolyzate [in the general formula (V), a is 186.9, b is 9.9, c is 82.2, d is 21.0 and a+b+c+d is 300] (400 g, the mole number of all anionic functional groups was 0.28 mol) was stirred with heating at 70°C, and, into this, 97.3 g of stearyl trimethylammonium chloride (100 mole% with respect to all anionic functional groups of the keratin hydrolyzate) was added, and the mixture was stirred with heating at 80°C until homogeneous.

By the above-described treatment, stearyl trimethylammonium was ionically bonded to all anionic functional groups of the keratin hydrolyzate to form an ion complex, to which water was added to adjust solid concentration to 40%. Thus, 493 g of an aqueous solution of the pea protein hydrolyzate was obtained, which has an ion complex of pea protein hydrolyzate with stearyl trimethylammonium, that is, all anionic functional groups was bonded to stearyl trimethylammonium and formed ion complexes, and has solid concentration of 40%.

Example 6

[Formation of ion complex of acylated derivative of soybean protein hydrolyzate (hereinafter, referred to as "acylated hydrolyzed soybean protein") with diethylaminoethylstearamide]

To 400 g of a 25% aqueous solution of a soybean protein hydrolyzate [in the general formula (V), a is 1.7, b is 0.3, c is 1.2, d is 0 and a+b+c+d is 3.2] (The mole number of all anionic functional groups was 0.54 mol. The mole number of amino groups was 0.25 mol, which was calculated based on measured values according to the above-described measurement method of the amino nitrogen and the total nitrogen amount. The mole number of amino groups was obtained by the same method also in examples below.) a 20% sodium hydroxide aqueous solution was added to adjust pH of the solution to 9. Coco-fatty acid chloride (58.8 g: one equivalent with respect to amino groups of the soybean protein hydrolyzate) was added dropwise into this solution over a period of 1 hour while stirring at 45°C and an acylation reaction was performed. During dropping of the coco-fatty acid chloride, a 20% NaOH aqueous solution was added to keep pH of the solution at 9. After completion of dropping, the mixture was stirred continuously at 50°C for 2 hours to complete the reaction. The acylation reaction rate of the above-described soybean protein hydrolyzate was 80.2%, which was a value calculated according to the following formula in which the amino nitrogen amount of the soybean protein hydrolyzate before the acylation reaction is represented by u1 and the amino nitrogen amount after the reaction is represented by t1.

$$\text{Acylation reaction rate (\%)} = 1\text{-}(t1/u1)\times 100$$

After completion of the reaction, dilute sulfuric acid was added to the reaction liquid to adjust pH to 2, thereby insolubilizing the acylated hydrolyzed soybean protein. This insoluble substance was washed with ion exchanged water repeatedly until pH became 3.5, thereby removing salts to obtain an acylated hydrolyzed soybean protein containing anionic functional groups under free condition (-COOH).

Next, this acylated hydrolyzed soybean protein solution was stirred with heating at 70°C, and, into this, diethylaminoethylstearamide (61.9 g: 30 mole% with respect to all anionic functional groups of the hydrolyzed soybean protein) was added, and the mixture was stirred with heating at 70°C until homogeneous.

By the above-described treatment, diethylaminoethylstearamide was ionically bonded to 30 mol% of anionic functional groups in all anionic functional groups of the hydrolyzed soybean protein to form an ion complex, to which water was added to adjust solid concentration to 40%. Thus, 432 g of an aqueous solution of the acylated hydrolyzed soybean protein was obtained, which has an ion complex with diethylaminoethylstearamide and has solid concentration of 40%.

Example 7

[Formation of ion complex of quaternized derivative of casein hydrolyzate (hereinafter, referred to as "quaternized hydrolyzed casein") with dimethylaminopropylstearamide (in the general formula (I), $R^1$ is a heptadecyl group, $R^2$ is a propylene group, $R^3$ is a methyl group and $R^4$ is a methyl group.)]

To 400 g of a 25% aqueous solution of a casein hydrolyzate [in the general formula (V), a is 3.6, b is 0.8, c is 1.8, d is 0 and a+b+c+d is 6.2] (The mole number of all anionic functional groups was 0.36 mol. The mole number of amino groups was 0.13 mol.), a 20% sodium hydroxide aqueous solution was added to adjust pH of the solution to 9. Glycidyl trimethylammonium chloride (19.7 g: one equivalent with respect to amino groups of the casein hydrolyzate) was added dropwise into this solution over a period of 1 hour while stirring at 55°C and a quaternization was performed. After completion of dropping, the mixture was stirred continuously at 55°C for 3 hours to complete the reaction to obtain quaternized hydrolyzed casein [N-[2-hydroxy-3-(trimethylammonio)propyl] hydrolyzed casein chloride]. The quaternized reaction rate of the above-described casein hydrolyzate was 76.6%, which was a value calculated according to the following formula in which the amino nitrogen amount of the casein hydrolyzate before the reaction is represented by u2 and the amino nitrogen amount after the reaction is represented by t2.

$$\text{Quaternized reaction rate (\%)} = 1\text{-}(t2/u2)\times 100$$

After completion of the reaction, dilute hydrochloric acid was added to the reaction solution of the above quaternized hydrolyzed casein to adjust pH to 2.5 and desalting was performed using an electrodialyzer to obtain an aqueous solution of quaternized hydrolyzed casein containing anionic functional groups under free condition (-COOH).

Next, this quaternized hydrolyzed casein solution, in which the anionic functional groups are under free condition, was stirred with heating at 70°C, and, into this, dimethylaminopropylstearamide (53.4 g: 50 mole% with respect to all anionic functional groups of the casein hydrolyzate) was added, and the mixture was stirred with heating at 70°C until homogeneous.

By the above-described treatment, dimethylaminopropylstearamide was ionically bonded to 50 mol% of anionic functional groups in all anionic functional groups of the quaternized hydrolyzed casein to form an ion complex. Then, the solution was concentrated to adjust solid concentration to 40%. Thus, 389 g of an aqueous solution of the quaternized hydrolyzed casein was obtained, which has an ion complex with dimethylaminopropylstearamide and has solid concentration of 40%.

Example 8

[Formation of ion complex of silylated derivative of sesame protein hydrolyzate (hereinafter, referred to as "silylated hydrolyzed sesame protein") with distearyl dimethylammonium(in the general formula (II), both $R^5$ are stearyl group.)]

To 400 g of a 25% aqueous solution of a sesame protein hydrolyzate [in the general formula (V), a is 5.6, b is 1.4, c is 3.0, d is 0 and a+b+c+d is 10] (The mole number of all anionic functional groups was 0.32 mol. The mole number

of amino groups was 0.08 mol.), a 20% sodium hydroxide aqueous solution was added to adjust pH of the solution to 9. Into this solution, 3-glycidoxypropylmethyldiethoxysilane (19.9 g: equivalent molar with respect to amino groups of the sesame protein hydrolyzate) was added dropwise over a period of 1 hour while stirring at 55°C and an silylation reaction was performed. After completion of dropping, the mixture was stirred continuously at 55°C for 3 hours to complete the reaction to obtain silylated hydrolyzed sesame protein [N-[2-hydroxy-3-(3-(dihydroxymethylsilyl) propoxy)propyl] hydrolyzed sesame protein]. The silylation reaction rate of the above-described sesame protein hydrolyzate was 72.0%, which was a value calculated according to the following formula in which the amino nitrogen amount of the sesame protein hydrolyzate before the reaction is represented by u3 and the amino nitrogen amount after the reaction is represented by t3.

$$\text{Silylation reaction rate (\%)} = 1\text{-}(t3/u3)\times 100$$

To the reaction liquid of silylated hydrolyzed sesame protein thus obtained, dilute hydrochloric acid was added to adjust pH to 3.0, and, then, distearyldimethylammonium chloride (56.2 g: 30 mole% with respect to all anionic functional groups of the sesame protein hydrolyzate) was added.

By the above-described treatment, distearyldimethylammonium was ionically bonded to 30 mol% of anionic functional groups in all anionic functional groups of the sesame protein hydrolyzate to form an ion complex. After cooling, the reaction liquid was left and the silylated sesame protein hydrolyzate having ion complex with distearyldimethylammonium was precipitated.

Next, this insolublized silylated sesame protein hydrolyzate having ion complex with distearyldimethylammonium was washed with ion exchanged water and dried to obtain 76.6 g of solid mass of silylated sesame protein hydrolyzate having ion complex with distearyldimethylammonium in which solid concentration was 92 %.

Example 9

[Formation of ion complex of glycerylated derivative of rice protein hydrolyzate (hereinafter, referred to as "glycerylated hydrolyzed rice protein") with behenyltrimethylammonium (in the general formula (II), one of $R^5$ is a behenyl group and other one is a methyl group)]

To 400 g of a 25% aqueous solution of a rice protein hydrolyzate [in the general formula (V), a is 62.5, b is 11.8, c is 25.4, d is 0.3 and a+b+c+d is 100] (The mole number of all anionic functional groups was 0.22 mol. The mole number of amino groups was 0.01 mol.) a 20% sodium hydroxide aqueous solution was added to adjust pH of the solution to 9. Glycidol (0.74 g: one equivalent with respect to amino groups of the rice protein hydrolyzate) was added dropwise into this solution over a period of 0.5 hour while stirring at 55°C and a glycerylation reaction was performed. After completion of dropping, the mixture was stirred continuously at 55°C for 3 hours to complete the reaction.

After completion of the reaction, dilute hydrochloric acid was added to the reaction liquid to adjust pH to 5. Then, de-salting was performed by an electrodialyzer to obtain glycerylated hydrolyzed rice protein. The glycerylation reaction rate of the above-described rice protein hydrolyzate was 70.8%, which was a value calculated according to the following formula in which the amino nitrogen amount of the rice protein hydrolyzate before the glycerylation reaction is represented by u4 and the amino nitrogen amount after the reaction is represented by t4.

$$\text{Glycerylation reaction rate (\%)} = 1\text{-}(t4/u4)\times 100$$

Next, this glycerylated hydrolyzed rice protein was stirred with heating at 80°C, and, into this, behenyltrimethylammonium chloride (88.8 g: 100 mole% with respect to all anionic functional groups of the rice protein hydrolyzate) was added, and the mixture was stirred with heating at 80°C.

By the above-described treatment, behenyltrimethylammonium was ionically bonded to all anionic functional groups of the glycerylated hydrolyzed rice protein to form an ion complex. After cooling, the reaction liquid was left and the ion complex was insolublized and precipitated.

Next, this insolublized substance was washed with ion exchanged water and dried to obtain 104 g of solid mass of ion complex of glycerylated hydrolyzed rice protein and behenyltrimethylammonium having solid concentration of 91%, that is, glycerylated hydrolyzed rice protein, having solid concentration of 91%, of which all anionic functional groups were formed ion complexes with behenyltrimethylammonium.

Example 10

[Formation of ion complex of alkylglycerylation derivative of silk hydrolyzate (hereinafter, referred to as "alkylglycerylated hydrolyzed silk") with diethylaminoethylstearamide]

To 400 g of a 25% aqueous solution of a silk hydrolyzate [in the general formula (V), a is 96.0, b is 0.9, c is 3.1, d is 0 and a+b+c+d is 100] (The mole number of all anionic functional groups was 0.05 mol. The mole number of amino groups was 0.013 mol.) a 20% sodium hydroxide aqueous solution was added to adjust pH of the solution to 9. Butylglycidyl ether (1.69 g: equivalent molar with respect to amino groups of the silk hydrolyzate) was added dropwise into this solution over a period of 1 hour while stirring at 55°C and an alkylglyceryIation reaction was performed. After completion of dropping, the mixture was stirred continuously at 55°C for 3 hours to complete the reaction to obtain alkylglycerylated hydrolyzed silk [that is, butylglycerylated hydrolyzed silk]. The alkylglycerylation reaction rate of the above-described silk hydrolyzate was 76.2 %, which was a value calculated according to the following formula in which the amino nitrogen amount of the silk hydrolyzate before the reaction is represented by u5 and the amino nitrogen amount after the reaction is represented by t5.
$$\text{Alkylglycerylation reaction rate (\%)} = 1\text{-}(t5/u5)\times 100$$

After completion of the reaction, dilute hydrochloric acid was added to the reaction liquid of above-described butylglycerylated hydrolyzed silk to adjust pH to 2.5. Then, de-salting was performed by an electrodialyzer to obtain an aqueous solution of butylglycerylated hydrolyzed silk containing anionic functional groups under free condition (-COOH).
Next, the aqueous solution of butylglycerylated hydrolyzed silk containing anionic functional groups under free condition was stirred with heating at 70°C, and, into this, 19.1 g of diethylaminoethylstearamide (100 mol% with respect to all anionic functional groups in the silk hydrolyzate.) was added, and the mixture was stirred with heating at 70°C until homogeneous.
By the above-described treatment, diethylaminoethylstearamide was ionically bonded to all anionic functional groups of the silk hydrolyzate to form an ion complex. Then, the solution was concentrated to adjust solid concentration to 40% to obtain 291 g of an aqueous solution of ion complex of butylglycerylated hydrolyzed silk and diethylaminoethylstearamide, that is, butylglycerylated hydrolyzed silk having solid concentration of 40%, of which all anionic functional groups were formed ion complexes with diethylaminoethylstearamide.

Example 11

[Formation of ion complex of 2-hydroxyalkyl derivative of collagen hydrolyzate (hereinafter, referred to as "2-hydroxyalkylated hydrolyzed collagen") with diethylaminoethylstearamide]

To 400 g of a 25% aqueous solution of a collagen hydrolyzate [in the general formula (V), a is 15.9, b is 1.7, c is 2.4, d is 0 and a+b+c+d is 20] (The mole number of all anionic functional groups was 0.17 mol. The mole number of amino groups was 0.05 mol.), a 20% sodium hydroxide aqueous solution was added to adjust pH of the solution to 9. Into this solution, 1,2-epoxyhexane (5.0 g: equivalent molar with respect to amino groups of the collagen hydrolyzate) was added dropwise over a period of 1 hour while stirring at 55°C and a 2-hydroxyalklation reaction was performed. After completion of dropping, the mixture was stirred continuously at 55°C for 3 hours to complete the reaction to obtain 2-hydroxyalkylated hydrolyzed collagen [that is, 2-hydroxyhexyl hydrolyzed collagen]. The 2-hydroxyalklation reaction rate of the above-described collagen hydrolyzate was 72.6 %, which was a value calculated according to the following formula in which the amino nitrogen amount of the collagen hydrolyzate before the reaction is represented by u6 and the amino nitrogen amount after the reaction is represented by t6.
$$\text{2-Hydroxyalklation reaction rate (\%)} = 1\text{-}(t6/u6)\times 100$$

After completion of the reaction, dilute hydrochloric acid was added to the reaction liquid of above-described 2-hydroxyhexyl hydrolyzed collagen to adjust pH to 2.5. Then, de-salting was performed by an electrodialyzer to obtain an aqueous solution of 2-hydroxyhexyl hydrolyzed collagen containing anionic functional groups under free condition (-COOH).

Next, the aqueous solution of 2-hydroxyhexyl hydrolyzed collagen containing anionic functional groups under free condition was stirred with heating at 70°C, and, into this, 64.9 g of diethylaminoethylstearamide (100 mol% with respect to all anionic functional groups in the collagen hydrolyzate.) was added, and the mixture was stirred with heating at 70°C until homogeneous.

By the above-described treatment, diethylaminoethylstearamide was ionically bonded to all anionic functional groups of the collagen hydrolyzate to form an ion complex. Then, the solution was concentrated to adjust solid concentration to 40% to obtain 403.5 g of an aqueous solution of ion complex of 2-hydroxyhexyl hydrolyzed collagen and diethylaminoethylstearamide, that is, 2-hydroxyhexyl hydrolyzed collagen having solid concentration of 40%, of which all anionic functional groups were formed ion complexes with diethylaminoethylstearamide.

Example 12

[Ion complex of polyaspartic acid with diethylaminoethylstearamide]

To 400 g of a 30% aqueous solution of polyaspartic acid [in the general formula (VI), e+f is 8, g is 0 and e+f+g is 8], hydrochloric acid was added to adjust pH of the solution to 2.0. Then, de-salting of the aqueous solution of polyaspartic acid was performed by an electrodialyzer to obtain an aqueous solution of polyaspartic acid containing anionic functional groups under free condition (-COOH).

Next, 400 g of an aqueous solution of polyaspartic acid (The mole number of all anionic functional groups was 0.88 mol.) having concentration of 25 % adjusted by adding water to the above aqueous solution of polyaspartic acid was stirred with heating at 70°C, and, into this, 100.8 g of diethylaminoethylstearamide (30 mol% with respect to all anionic functional groups in the polyaspartic acid) was added, and the mixture was stirred with heating at 70°C until homogeneous.

By the above-described treatment, diethylaminoethylstearamide was ionically bonded to 30 mole% of all anionic functional groups of the polyaspartic acid to form ion complex. Then, the solution was concentrated to adjust the solid concentration to 40% to obtain 474 g of an aqueous solution of ion complex of polyaspartic acid with diethylaminoethylstearamide having solid concentration of 40%.

Example 13

[Ion complex of polyglutamic acid with sytearyltrimethylammoniumchloride]

To 400 g of a 25 % aqueous solution of polyglutamic acid [in the general formula (VI), e+f is 0, g is 20 and e+f+g is 20], hydrochloric acid was added to adjust pH of the solution to 4.0. Then, de-salting of the aqueous solution of polyglutamic acid was performed by an electrodialyzer to obtain an aqueous solution of de-salted polyglutamic acid.

Next, into the aqueous solution of polyglutamic acid, 205.7 g of stearyl trimethylammonium chloride (80 mol% with respect to all anionic functional groups in the polyglutamic acid) was added, and the mixture was stirred with heating at 70°C until homogeneous.

By the above-described treatment, stearyl trimethylammonium was ionically bonded to 80 mole% of all anionic functional groups of the polyglutamic acid to form ion complex. Then, solid concentration of the solution was adjustded to 40% to obtain 726 g of an aqueous solution of polyglutamic acid containing ion complex with stearyl trimethylammonium having solid concentration of 40%.

Examples 14-16, Comparative Examples 1-3 and Control Example 1: Hair treatment

Hair treatments having the composition shown in Table 1 were prepared as Examples 14-16 using the ion complex of a wheat protein hydrolyzate and an aliphatic amide amine formed in Examples 1-3 (that is, wheat protein hydrolyzate having an ion complex with an aliphatic amide amine). Hair treatments of Comparative Examples 1-3 were prepared likewise in which the wheat protein hydrolyzate and the aliphatic amide amine used as raw materials in forming an ion complex in Example 1 were compounded without forming an ion complex, instead of the wheat protein hydrolyzate having an ion complex formed in Example 1, 2 or3. Also a hair treatment was prepared as Control Example 1 in which none of the wheat protein hydrolyzate having an ion complex, the wheat protein hydrolyzate not forming an ion complex and aliphatic amide amine was compounded. In showing the compositions of the hair treatments of Examples 14-16, Comparative Examples 1 to 3 and Control Example 1 in Table 1, components compounded in common in the hair treatments are shown in the following Table, as a hair treatment base agent, and the total amount of these common components is shown as the amount of the hair treatment base agent in Table 1, from the standpoint of space. The compounding amounts of the compounding components are expressed by parts by mass in all cases.

Hair treatment base agent:

| | |
|---|---|
| Liquid paraffin #70S | 1.0 |
| Cetyl alcohol | 1.5 |
| Stearyl alcohol | 1.0 |
| Sytearyltrimethylammoniumchloride | 2.8 |
| Methylpolysiloxane 100cs | 2.0 |
| Phenoxyethanol | 0.5 |
| Total: | 8.8 |

(Table 1)

| Component | Example 14 | Example 15 | Example 16 | Comparative Example 1 | Comparative Example 2 | Comparative Example 3 | Control Example 1 |
|---|---|---|---|---|---|---|---|
| Hair treatment base agent | 8.8 | 8.8 | 8.8 | 8.8 | 8.8 | 8.8 | 8.8 |
| Wheat protein hydrolyzate having an ion complex of Example 1 (38%) | 3.0 | - | - | - | - | - | - |
| Wheat protein hydrolyzate having an ion complex of Example 2 (38%) | - | 3.0 | - | - | - | - | - |
| Wheat protein hydrolyzate having an ion complex of Example 3 (38%) | - | - | 3.0 | - | - | - | - |
| Wheat protein hydrolyzate used for forming ion complexes in Examples 1- 3 | - | - | - | 0.62 | 0.62 | 0.62 | - |
| Diethylaminoethylstearamide used for forming an ion complex in Example 1 | - | - | - | 0.52 | - | - | - |
| A mixture of diethylaminoethyl stearamide, diethylaminoethyl behenamide and diethylaminoethyl isostearamide used for forming an ion complex in Example 2 | - | - | - | - | 0.52 | - | - |
| A mixture of diethylaminoethyl stearamide, diethylaminoethyl behenamide and diethylaminoethyl isostearamide used for forming an ion complex in Example 3 | - | - | - | - | - | 0.52 | - |
| Purified water | Amount adjusted to 100 parts by mass | | | | | | |

The hair treatments prepared in Examples 14-16, Comparative Examples 1-3 and Control Example 1, as described above, were used in [Treatment with hair treatment] shown below on a bundle of hair damaged by [Bleach treatment] shown below, and subjected to [Sensory test]. The results are shown in Table 2.

[Bleach treatment]

Hair bundles having a length of 15 cm and a weight of 1.5 g were immersed in an aqueous solution containing 3% hydrogen peroxide water and 1% ammonia at 30°C for 30 minutes, then, washed with tap water. This treatment was repeated five times, as the bleach treatment.

[Treatment with hair treatment]

The hair bundles after the above-described bleach treatment were washed with a 2% sodium polyoxyethylene (3) lauryl ether sulfate aqueous solution. Each hair bundle was treated using each 2 g of the hair treatments of Examples 14-16, Comparative Examples 1-3 and Control Example 1 described above, then, rinsed with flowing water and dried by a hair drier. Washing with the sodium polyoxyethylene(3) lauryl ether sulfate aqueous solution, treatment with the hair treatment preparation, rinsing with flowing water and drying by a drier were repeated five times.

[Sensory test]

The hair bundles treated with the hair treatments of Examples 14-16, Comparative Examples 1-3 and Control Example 1, as described above, were evaluated under blind conditions (blind trial) by 10 panelists for four items: flexibility, moisture retaining feeling, smoothness and luster. Based on Control Example 1, the point evaluated was <2> when better than Control Example 1, evaluated was <0> when worse than Control Example 1 and evaluated was <1> when equivalent to Control Example 1. The total point of every evaluation items was used for evaluation.
The results of this [Sensory test] are shown in Table 2 below. Further, appearances of the prepared hair treatments as an emulsifying product are also described together in Table 2.
(Table 2)

| | Example 14 | Example 15 | Example 16 | Comparative Example 1 | Comparative Example 2 | Comparative Example 3 |
|---|---|---|---|---|---|---|
| Flexibility | 15 | 16 | 18 | 10 | 10 | 9 |
| Moisture retaining property | 18 | 18 | 20 | 10 | 11 | 14 |
| Smoothness | 20 | 18 | 17 | 14 | 15 | 15 |
| Luster | 18 | 16 | 17 | 10 | 12 | 10 |
| Appearance of Hair treatment | Uniform emulsifying product | Uniform emulsifiying product | Uniform emulsifying product | Nonuniform emulsifying product | Nonuniform emulsifying product | Nonuniform emulsifying product |

As shown in Table 2, the hair treatments of Examples 14-16 containing the wheat protein hydrolyzate having an ion complex of Example 1, 2 or 3 showed better sensory characters in all evaluation items, as compared with the hair treatments of Comparative Examples 1 to 3 in which a wheat protein hydrolyzate and an aliphatic amide amine were compounded without forming an ion complex. Namely, the wheat protein hydrolyzate having an ion complex with an aliphatic amide amine of Examples 1-3 could be confirmed as a cosmetic base material which is adsorbed on hair and is capable of imparting flexibility, moisture retaining feeling, smoothness and luster to hair. The hair treatments of Examples 14-16 was a uniform emulsifying product while the hair treatments of Comparative Examples 1 to 3 were nonuniform emulsifying products. Thus, it was apparent that the cosmetic base material composed of the wheat protein hydrolyzate having an ion complex of Example 1, 2 or 3 was easily compounded in an emulsifying cosmetic such as a hair treatment and the like.

Examples 17-19, Comparative Examples 4-6 and Control Example 2: Shampoo

Shampoos having the composition shown in Table 3 were prepared as Examples 17-19 using the ion complex of a pea protein hydrolyzate and a mixture of aliphatic amide amines formed in Example 4 (that is, pea protein hydrolyzate having an ion complex with a mixture of aliphatic amide amines ), the ion complex of a keratin hydrolyzate and stearyltrimethylammonium chloride formed in Example 5 (that is, keratin hydrolyzate having an ion complex with stearyl trimethylammonium chloride), and the ion complex of a acylated hydrolyzed soybean protein with diethylaminoethylstearamide formed in Example 6 (that is, acylated hydrolyzed soybean protein having an ion complex with diethylaminoethylstearamide). A shampoo of Comparative Example 4 was prepared likewise in which the pea protein hydrolyzate and the mixture of aliphatic amide amines used as raw materials in forming an ion complex in Example 4 were compounded without forming an ion complex, instead of the pea protein hydrolyzate having an ion complex formed in Example 4. A shampoo of Comparative Example 5 was prepared likewise in which the keratin hydrolyzate and the stearyl trimethylammonium chloride used as raw materials in forming an ion complex in Example 5 were compounded without forming an ion complex, instead of the keratin hydrolyzate having an ion complex formed in Example 5. A shampoo of Comparative Example 6 was prepared likewise in which the acylated hydrolyzed soybean protein and the diethylaminoethylstearamide used as raw materials in forming an ion complex in Example 6 were compounded without forming an ion complex, instead of the acylated hydrolyzed soybean protein having an ion complex formed in Example 6. Also a shampoo was prepared as Control Example 2 in which none of the pea protein hydrolyzate, keratin hydrolyzate, acylated hydrolyzed soybean protein, aliphatic amide amine and stearyl trimethylammonium chloride were compounded. In showing the compositions of the shampoo of Examples 17-19, Comparative Examples 4 - 6 and Control Example 2 in Table 3,components compounded in common in the shampoos are shown in the following Table, as a shampoo base agent, and the total amount of these common components is shown as the amount of the shampoo base agent in Table 3, from the standpoint of space. In addition, from the standpoint of space, in Table 3, "the component used for forming an ion complex in Example 4" was expressed as "component used in Example 4" for simplification, "the component used for forming an ion complex in Example 5" was expressed as "component used in Example 5" for simplification, and "the component used for forming an ion complex in Example 6" was expressed as "component used in Example 6" for simplification. The compounding amounts of the compounding components are expressed by parts by mass in all cases.

Sampoo base agent:

| | |
|---|---|
| Sodium polyoxyethlene(3)laurylether sulfate(30%) | 30.0 |
| Coco-fatty acid N-methylethanol amide*1 | 5.0 |
| Cationized cellulose*2 | 0.1 |
| A mixture of para-oxybenzoic acid ester and Phenoxyethanol*3 | 0.5 |
| Total: | 35.6 |

*1 COCAMID METHYL MEA (commercial name); manufactured by Kao Cooporation
*2 Ucare Polymer JR-400 (commercial name); manufactured by Union Carbide Corporation
*3 Seisept-H (commercial name); manufactured by Seiwa Kasei Co., Ltd.

(Table 3)

| Component | Example 17 | Example 18 | Example 19 | Comparative Example 4 | Comparative Example 5 | Comparative Example 6 | Control Example 2 |
|---|---|---|---|---|---|---|---|
| Sampoo base agent | 35.6 | 35.6 | 35.6 | 35.6 | 35.6 | 35.6 | 35.6 |
| Pea protein hydrolyzate having an ion complex of Example 4 (40%) | 5.0 | - | - | - | - | - | - |
| Keratin hydrolyzate having an ion complex of Example 5 (40%) | - | 5.0 | - | - | - | - | - |
| Acylated hydrolyzed soybean protein having an ion complex of Example 6 (40%) | - | - | 5.0 | - | - | - | - |
| Pea protein hydrolyzate used in Example 4 | - | - | - | 1.2 | - | - | - |
| A mixture of diethylaminoethyl stearamide, diethylaminoethyl behenamide and diethylaminoethyl isostearamide used in Example 4 | - | - | - | 0.8 | - | - | - |
| Keratin protein hydrolyzate used in Example 5 | - | - | - | - | 1.0 | - | - |
| Stearyldimethylammonium chloride used in Example 5 | - | - | - | - | 1.0 | - | - |
| Acylated hydrolyzed soybean protein used in Example 6 | - | - | - | - | - | 1.4 | - |
| Diethylaminoethylstearamide used in Example 6 | - | - | - | - | - | 0.6 | - |
| Purified water | Amount adjusted to 100 parts by mass | | | | | | |

The hair bundle after the above-described bleach treatment was washed with 2g of shampoo prepared in Examples 17-19, Comparative Examples 4-6 or Control Example 2, as described above, then, rinsed with flowing water and dried by a hair drier. Washing with the shampoo, rinsing with flowing water and drying by a drier were repeated ten times. Then, the hair bundle was subjected to [Sensory test] same as that described above. The results are shown in Table 4.

(Table 4)

| | Example 17 | Example 18 | Example 19 | Comparative Example 4 | Comparative Example 5 | Comparative Example 6 |
|---|---|---|---|---|---|---|
| Flexibility | 15 | 15 | 16 | 10 | 10 | 11 |
| Moisture retaining property | 17 | 18 | 18 | 11 | 11 | 15 |
| Smoothness | 19 | 18 | 19 | 14 | 15 | 14 |
| Luster | 16 | 18 | 18 | 10 | 11 | 10 |

As shown in Table 4, the shampoos of Examples 17-19 containing the peptide or peptide derivative having an ion complex of Example 4, 5 or 6 showed better sensory characters in all evaluation items, as compared with the shampoos of Comparative Examples 4 to 6 in which the peptide or peptide derivative and aliphaticamideamine or alkyl type quaternary ammonium were compounded without forming an ion complex. Namely, the the peptide or peptide derivative having an ion complex of Examples 4-6 could be confirmed as a cosmetic base material which is adsorbed on hair and is capable of imparting flexibility, moisture retaining feeling, smoothness and luster to hair.

Examples 20-22, Comparative Examples 7-9 and Control Example 3: Permanent wave first agent

Permanent wave first agents having the composition shown in Table 5 were prepared as Examples 20-22 using the ion complex of quaternized hydrolyzed casein and dimethylaminopropylstearamide formed in Example 7 (that is, quaternized hydrolyzed casein having an ion complex with dimethylaminopropylstearamide), the ion complex of a silylated hydrolyzed sesame protein and distearyldimethylammonium chloride formed in Example 8 (that is, a silylated hydrolyzed sesame protein having an ion complex with distearyldimethylammonium chloride), and the ion complex of a glycerylated hydrolyzed rice protein and behenyltrimethylammonium chloride formed in Example 9 (that is, glycerylated hydrolyzed rice protein having an ion complex with behenyltrimethylammonium chloride). A permanent wave first agent of Comparative Example 7 was prepared likewise in which the quaternized hydrolyzed casein and the dimethylaminopropylstearamide used as raw materials in forming an ion complex in Example 7 were compounded without forming an ion complex, instead of the quaternized hydrolyzed casein having an ion complex formed in Example 7. A permanent wave first agent of Comparative Example 8 was prepared likewise in which the silylated hydrolyzed sesame protein and the distearyldimethylammonium chloride used as raw materials in forming an ion complex in Example 8 were compounded without forming an ion complex, instead of the silylated hydrolyzed sesame protein having an ion complex formed in Example 8. A permanent wave first agent of Comparative Example 9 was prepared likewise in which the glycerylated hydrolyzed rice protein and the behenyltrimethylammonium used as raw materials in forming an ion complex in Example 9 were compounded without forming an ion complex, instead of the glycerylated hydrolyzed rice protein having an ion complex formed in Example 9. Also a permanent wave first agent was prepared as Control Example 3 in which none of the quaternized hydrolyzed casein, silylated hydrolyzed sesame protein, glycerylated hydrolyzed rice protein, dimethylaminopropylstearamide, distearyldimethylammonium chloride, behenyltrimethylammonium chloride were compounded. In showing the compositions of the permanent wave first agents of Examples 20-22, Comparative Examples 7- 9 and Control Example 3 in Table 5, components compounded in common in the permanent wave first agents are shown in the following Table, as a base agent of permanent wave first agent, and the total amount of these common components is shown as the amount of the base agent of permanent wave first agent in Table 5, from the standpoint of space. In addition, from the standpoint of space, in Table 5, "the component used for forming an ion complex in Example 7" was expressed as "component used in Example 7" for simplification, "the component used for forming an ion complex in Example 8" was expressed as "component used in Example 8" for simplification, and "the component used for forming an ion complex in Example 9" was expressed as "component used in Example 9" for simplification. The compounding amounts of the compounding components are expressed by parts by mass in all cases.

Base agent of permanent wave first agent:

| | |
|---|---|
| Ammonium thioglycolate(50%) | 12.0 |
| Monoethanolamine | 1.8 |
| Polyoxyethlene (15) laurylether | 0.5 |
| Disodium edetate | 0.1 |
| Aqueous ammonia(28%) | 1.6 |
| | Total: 16.0 |

| Component | Example 20 | Example 21 | Example 22 | Comparative Example 7 | Comparative Example 8 | Comparative Example 9 | Control Example 3 |
|---|---|---|---|---|---|---|---|
| Base agent of permanent wave first agent | 16.0 | 16.0 | 16.0 | 16.0 | 16.0 | 16.0 | 16.0 |
| Quaternized hydrolyzed casein having an ion complex of Example 7 (40%) | 2.0 | - | - | - | - | - | - |
| Silylated hydrolyzed sesame protein having an ion complex of Example 8 (92%) | - | 0.9 | - | - | - | - | - |
| Glycerylated hydrolyzed rice protein having an ion complex of Example 9 (91%) | - | - | 0.9 | - | - | - | - |
| Quaternized hydrolyzed casein used in Example 7 | - | - | - | 0.55 | - | - | - |
| Dimethylaminopropylstearamide used in Example 7 | - | - | - | 0.25 | - | - | - |
| Silylated hydrolyzed sesame protein used in Example 8 | - | - | - | - | 0.54 | - | - |
| Distearyldimethylammonium chloride used in Example 8 | - | - | - | - | 0.26 | - | - |
| Glycerylated hydrolyzed rice protein used in Example 9 | - | - | - | - | - | 0.43 | - |
| Behenyltrimethylammonium chloride used in Example 9 | - | - | - | - | - | 0.37 | - |
| Purified water | Amount adjusted to 100 parts by mass | | | | | | |

Treatment for hair with the permanent wave first agent prepared in Examples 20-22, Comparative Examples 7-9 or Control Example 3, as described above, was conducted as follows. That is, hairs having the same length of 20 cm were rinsed with 2% aqueous solution of sodium polyoxyethylene(3)laurylether sulfate, and, then, rinsed with running tap water, followed by being dried in air. Seven bundles each consisting of 50 hairs were prepared from the hairs obtained above, and each of them was wound on a rod having length of 10cm and diameter of 1cm. On the hair bundle wound on the rod, 2ml of the permanent wave first agent of Examples 20-22, Comparative Examples 7-9 or Control Example 3 was applied, and, then, the bundle was wrapped with plastic film. Thereafter, the bundle was left as it was for 15 minutes, followed by rinsed gently with running tap water for 10 seconds. Then, 2ml of a permanent wave second agent was applied, and, the bundle was wrapped with plastic film. Thereafter, the bundle was left as it for 15 minutes, followed by rinsing gently with running tap water. Each bundle was dried in a hot air drier at 60°C. After dried, the hair bundle was subjected to [Sensory test] same as that described above. The results are shown in Table 6.

(Table 6)

| | Example 20 | Example 21 | Example 22 | Comparative Example 7 | Comparative Example 8 | Comparative Example 9 |
|---|---|---|---|---|---|---|
| Flexibility | 20 | 18 | 16 | 12 | 10 | 10 |
| Moisture retaining property | 20 | 18 | 17 | 14 | 11 | 13 |
| Smoothness | 18 | 20 | 19 | 15 | 15 | 14 |
| Luster | 16 | 20 | 19 | 12 | 11 | 10 |

As shown in Table 6, the permanent wave first agents of Examples 20-22 containing the peptide derivative having an ion complex of Example 7, 8 or 9 showed better sensory characters in all evaluation items, as compared with the permanent wave first agents of Comparative Examples 7-9 in which the peptide derivative and aliphatic amideamine or alkyl type quaternary ammonium were compounded without forming an ion complex. Namely, the peptide derivative having an ion complex of Example 7, 8 or 9 could be confirmed as a cosmetic base material which is adsorbed on hair and is capable of imparting flexibility, moisture retaining feeling, smoothness and luster to hair.

Examples 23-25, Comparative Examples 10-12 and Control Example 4: Oxidation type hair dye first agent

Oxidation type hair dye first agents having the composition shown in Table 7 were prepared as Examples 23-25 using the ion complex of a butylglycerylated hydrolyzed silk and diethylaminoethylstearamide formed in Example 10 (that is, butylglycerylated hydrolyzed silk having an ion complex with diethylaminoethylstearamide), the ion complex of a 2-hydroxyhexyl hydrolyzed collagen and diethylaminoethylstearamide formed in Example 11 (that is, a 2-hydroxyhexyl hydrolyzed collagen having an ion complex with diethylaminoethylstearamide), and the ion complex of a polyaspartic acid and diethylaminoethylstearamide formed in Example 12 (that is, polyaspartic acid having an ion complex with diethylaminoethylstearamide). An oxidation type hair dye first agent of Comparative Example 10 was prepared likewise in which the butylglycerylated hydrolyzed silk and the diethylaminoethylstearamide used as raw materials in forming an ion complex in Example 10 were compounded without forming an ion complex, instead of the butylglycerylated hydrolyzed silk having an ion complex formed in Example 10. An oxidation type hair dye first agent of Comparative Example 11 was prepared likewise in which the 2-hydroxyhexyl hydrolyzed collagen and the diethylaminoethylstearamide used as raw materials in forming an ion complex in Example 11 were compounded without forming an ion complex, instead of the 2-hydroxyhexyl hydrolyzed collagen having an ion complex formed in Example 11. An oxidation type hair dye first agent of Comparative Example 12 was prepared likewise in which the polyaspartic acid and the diethylaminoethylstearamide used as raw materials in forming an ion complex in Example 12 were compounded without forming an ion complex, instead of the polyaspartic acid having an ion complex formed in Example 12. Also an oxidation type hair dye first agent was prepared as Control Example 4 in which none of the butylglycerylated hydrolyzed silk, 2-hydroxyhexyl hydrolyzed collagen, polyaspartic acid and diethylaminoethylstearamide were compounded. In showing the compositions of the oxidation type hair dye first agents of Examples 23-25, Comparative Examples 10-12 and Control Example 4, components compounded in common in the oxidation type hair dye first agents are shown in the following Table as the base agent of oxidation type hair dye first agent, and the total amount of these common components is shown as the amount of the base agent of oxidation type hair dye first agent in Table 7, from the standpoint of space. In addition, from the standpoint of space, in Table 7, "the component used for forming an ion complex in Example 10" was expressed as "component

used in Example 10" for simplification, "the component used for forming an ion complex in Example 11" was expressed as "component used in Example 11" for simplification, and "the component used for forming an ion complex in Example 12" was expressed as "component used in Example 12" for simplification. The compounding amounts of the compounding components are expressed by parts by mass in all cases.

Base agent of oxidation type hair dye first agent:

| | |
|---|---|
| p-Phenylene diamine | 3.0 |
| Resorcin | 0.5 |
| Oleic acid | 20.0 |
| Polyoxyethlene (10) oleylether | 15.0 |
| Isopropanol | 10.0 |
| Aqueous ammonia (28%) | 10.0 |
| | Total: 58.5 |

(Table 7)

| Component | Example 23 | Example 24 | Example 25 | Comparative Example 10 | Comparative Example 11 | Comparative Example 12 | Control Example 4 |
|---|---|---|---|---|---|---|---|
| Base agent of oxidation type hair dye first agent | 58.5 | 58.5 | 58.5 | 58.5 | 58.5 | 58.5 | 58.5 |
| Butylglycerylated hydrolyzed silk having an ion complex of Example 10 (40%) | 1.0 | | - | - | - | - | - |
| 2-Hydroxyhexyl hydrolyzed collagen having an ion complex of Example 11 (40%) | - | 1.0 | - | - | - | - | - |
| Polyaspartic acid having an ion complex of Example 12 (40%) | - | - | 1.0 | - | - | - | - |
| Butylglycerylated hydrolyzed silk used in Example 10 | | - | | 0.2 | - | - | - |
| 2-Hydroxyhexyl hydrolyzed collagen used in Example 11 | - | - | - | - | 0.33 | - | - |
| Polyaspartic acid used in Example 12 | | - | - | - | - | 0.25 | - |
| Diethylaminoethylstearamide used in Examples 10-12 | - | - | - | 0.2 | 0.07 | 0.15 | - |
| Purified water | Amount adjusted to 100 parts by mass | | | | | | |

Compositions of the oxidation type hair dye second agents used in combination with the oxidation type hair dye first agents of Examples 23-25, Comparative Examples 10- 12 and Control Example 4 are shown in Table 8.
(Table 8)

| Component | Composition |
|---|---|
| Stearic acid | 1.0 |
| Glyceryl monostearate | 1.5 |
| Polyoxyethlene (10) oreylether | 1.0 |
| Aqueous hydroperoxide (35%) | 17.0 |
| Purified water | Amount adjusted to 100 |

Treatment for hair with the oxidation type hair dye first agent and second agent was conducted as follows. That is, seven bundles of hair, each bundle being the length of 15 cm and the weight of 1 g; were prepared. Each bundle was washed with 2% aqueous solution of sodium polyoxyethylene(3)laurylether sulfate, and, then, rinsed with running tap water, followed by being dried in air. On each hair bundle obtained above, 2g of oxidation type hair dye, being prepared by mixing each of the oxidation type hair dye first agent of Examples 23-25, Comparative examples 10-12 or Control Example 5 with the equivalent amounts of oxidation type hair dye second agent shown in Table 8, was applied uniformly on each hair bundle. Then, the hair bundle was left as it was for 30 minutes, rinsed with hot water. Thereafter, the hair bundle was washed with 2% aqueous solution of sodium polyoxyethylene(3)laurylether sulfate, and, then, rinsed with running tap water, followed by being dried by hot air with a hair drier. After dried, the hair bundle was subjected to [Sensory test] same as that described above. The results are shown in Table 9.
(Table 9)

| | Example 23 | Example 24 | Example 25 | Comparative Example 10 | Comparative Example 11 | Comparative Example 12 |
|---|---|---|---|---|---|---|
| Flexibility | 17 | 16 | 16 | 12 | 11 | 11 |
| Moisture retaining property | 20 | 20 | 19 | 15 | 14 | 14 |
| Smoothness | 17 | 17 | 18 | 14 | 13 | 13 |
| Luster | 15 | 16 | 17 | 12 | 11 | 11 |

As shown in Table 9, the oxidation type hair dye first agents of Examples 23-25 containing the peptide or peptide derivative having an ion complex of Example 10, 11 or 12 showed better sensory characters in all evaluation items, as compared with the oxidation type hair dye first agents of Comparative Examples 10-12 in which the peptide or peptide derivative and diethylaminoethylstearamide were compounded without forming an ion complex. Namely, the peptide or peptide derivative having an ion complex of Example 10, 11 or 12 could be confirmed as a cosmetic base material which is adsorbed on hair and is capable of imparting flexibility, moisture retaining feeling, smoothness and luster to hair.

**Claims**

1. A cosmetic base material composed of a peptide or peptide derivative having one or more acidic amino acid as the constituent amino acid and in which at least some of anionic functional groups thereof form an ion complex with at least one surfactant selected from the group consisting of cationic surfactants and ampholytic surfactants.

2. The cosmetic base material according to Claim 1 in which 30 mol% or more of all anionic functional groups in the above-described peptide or peptide derivative having one or more acidic amino acid as the constituent amino acid are ionically bonded to at least one surfactant selected from the group consisting of cationic surfactants and ampholytic surfactants to form an ion complex.

3. The cosmetic base material according to Claim 1 or 2 in which the above-described at least one surfactant selected from the group consisting of cationic surfactants and ampholytic surfactants includes a fatty acid amide amine represented by the general formula (I):

(Chemical formula 1)

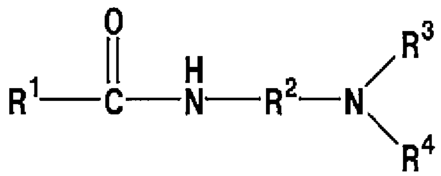

(I)

(wherein, $R^1$ represents a linear or branched hydrocarbon group having 11 to 25 carbon atoms, $R^2$ represents an alkylene group having 1 to 3 carbon atoms, and $R^3$ and $R^4$ represent an alkyl group having 1 to 3 carbon atom.) and/or an alkyl type quaternary ammonium represented by the general formula (II):

(Chemical formula 2)

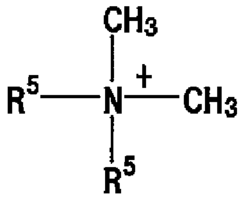

(II)

(wherein, $R^5$ represents a methyl group or a linear or branched hydrocarbon group having 11 to 25 carbon atoms, providing that at least one of two $R^5$s is a linear or branched hydrocarbon group having 11 to 25 carbon atoms.).

4. The cosmetic base material according to any one of Claims 1 to 3 wherein the average degree of amino acid polymerization of a peptide portion of the above-described peptide or peptide derivative is 2 to 500.

5. The cosmetic base material according to any one of Claims 1 to 4 wherein the above-described peptide or peptide derivative is a protein hydrolyzate, a derivative of a protein hydrolyzate, a polyacidic amino acid or a derivative of a polyacidic amino acid.

6. The cosmetic base material according to any one of Claims 1 to 4 wherein the peptide portion of the peptide or peptide derivative having an acidic amino acid is a hydrolyzate of a plant protein or a keratin hydrolyzate.

7. The cosmetic base material according to any one of Claims 1 to 4 wherein the peptide derivative is an acylated hydrolyzed protein, a glycerylated hydrolyzed protein, a quaternized hydrolyzed protein, a silylated hydrolyzed protein, an alkyl glycerylated hydrolyzed protein or a 2-hydroxyalkylated hydrolyzed protein.

8. A cosmetic comprising the cosmetic base material according to any one of Claims 1 to 7.

9. The cosmetic according to Claim 8 wherein the content of the above-described cosmetic base material is 0.1 to 30 % by mass.

**INTERNATIONAL SEARCH REPORT**

International application No.
PCT/JP2011/059045

A. CLASSIFICATION OF SUBJECT MATTER
*A61K8/64*(2006.01)i, *A61K8/40*(2006.01)i, *A61K8/42*(2006.01)i, *A61Q5/02* (2006.01)i, *A61Q5/04*(2006.01)i, *A61Q5/10*(2006.01)i, *A61Q5/12*(2006.01)i

According to International Patent Classification (IPC) or to both national classification and IPC

B. FIELDS SEARCHED

Minimum documentation searched (classification system followed by classification symbols)
A61K8/64, A61K8/40, A61K8/42, A61Q5/02, A61Q5/04, A61Q5/10, A61Q5/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | JP 2009-161519 A (Seiwa Kasei Co., Ltd.), 23 July 2009 (23.07.2009), claims 1, 3 to 7; examples 1, 3, 10, 12 (Family: none) | 1-9 |
| X | JP 1-204998 A (Kobayashi Pharmaceutical Co., Ltd.), 17 August 1989 (17.08.1989), claims 1, 2, 4, 5; page 2, lower right column, lines 4 to 13; page 3, upper right column, lines 1 to 8 (Family: none) | 1-5,8,9 |
| E,X | JP 2011-88828 A (Seiwa Kasei Co., Ltd.), 06 May 2011 (06.05.2011), claims 1 to 9 (Family: none) | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 July, 2011 (12.07.11) | 26 July, 2011 (26.07.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/059045

C (Continuation). DOCUMENTS CONSIDERED TO BE RELEVANT

| Category* | Citation of document, with indication, where appropriate, of the relevant passages | Relevant to claim No. |
|---|---|---|
| E,X | JP 2011-84489 A (Seiwa Kasei Co., Ltd.), 28 April 2011 (28.04.2011), claims 1, 4 to 6; examples 8, 12, 14 (Family: none) | 1-9 |
| A | WO 2010/110455 A1 (Kagoshima University), 30 September 2010 (30.09.2010), claims 1, 3 (Family: none) | 1-9 |
| A | JP 2008-255046 A (Toray Industries, Inc.), 23 October 2008 (23.10.2008), claims 1, 5 (Family: none) | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007302615 A
- JP 2004196733 A
- JP 2004231517 A
- JP 6045526 B
- JP 4247805 B
- JP 3981828 B
- JP 2005306799 A
- JP 8059424 A
- JP 8067608 A